# EUROPEAN PATENT APPLICATION

(11) **EP 4 450 471 A1**
(43) Date of publication of application: **23.10.2024**
(21) Application number: 22907413.3
(22) Date of filing: 12.12.2022
(51) Int. Cl.: C03C 27/12

(54) **INTERMEDIATE FILM FOR LAMINATED GLASS AND LAMINATED GLASS**

(30) Priority: 13.12.2021 JP 2021201779
(71) Applicant: SEKISUI CHEMICAL CO., LTD., Osaka-shi Osaka 530-8565 (JP)
(72) Inventor: TAKAI, Minako, Kouka-shi, Shiga 528-8585 (JP); NOHARA, Atsushi, Kouka-shi, Shiga 528-8585 (JP)
(74) Representative: Ter Meer Steinmeister & Partner
(86) International application number: PCT/JP2022/045660
(87) International publication number: WO 2023/112889

(57) **Abstract**

Provided is an interlayer film for laminated glass capable of making yellowing less likely to occur, and enhancing the adhesive force between the interlayer film and a lamination glass member. An interlayer film for laminated glass according to the present invention is an interlayer film for laminated glass having a one-layer or two or more-layer structure, and contains an ultraviolet absorber represented by formula (X), and a metal salt: wherein R₁ represents any group, R₂ to R₈ each represent a hydrogen atom, an atom other than hydrogen atom, or any group.

## Description

### TECHNICAL FIELD

The present invention relates to an interlayer film for laminated glass that is used for obtaining laminated glass. Also, the present invention relates to laminated glass.

### BACKGROUND ART

Since laminated glass generates only a small amount of scattering glass fragments even when subjected to external impact and broken, laminated glass is excellent in safety. As such, laminated glass is widely used for automobiles, railway vehicles, aircraft, ships, buildings and the like. Laminated glass is produced by sandwiching an interlayer film between a pair of glass plates.

In order to suppress transmission of ultraviolet rays, an interlayer film containing an ultraviolet absorber having a benzotriazole skeleton is sometimes used (for example, Patent Document 1 below). Further, in laminated glass, an interlayer film containing a metal salt is sometimes used so as to enhance the adhesive force between the interlayer film and a lamination glass member (glass plate and the like).

### Related Art Document

### Patent Document

Patent Document 1: WO2015/088866 A1

### SUMMARY OF THE INVENTION

### PROBLEMS TO BE SOLVED BY THE INVENTION

In order to suppress transmission of ultraviolet rays, and enhance the adhesive force between an interlayer film and a lamination glass member, it is conceivable to use an interlayer film containing both an ultraviolet absorber and a metal salt. However, with a combination of a conventional ultraviolet absorber having a benzotriazole skeleton, and a metal salt, yellowing sometimes occur in the interlayer film. Although the yellowing can be suppressed to some extent by reducing the content of the metal salt, the adhesive force between the interlayer film and a lamination glass member deteriorates.

Therefore, in a conventional interlayer film containing an ultraviolet absorber having a benzotriazole skeleton, and a metal salt, it is difficult to exert both the effect of making yellowing less likely to occur, and the effect of enhancing the adhesive force between the interlayer film and a lamination glass member.

It is an object of the present invention to provide an interlayer film for laminated glass capable of making yellowing less likely to occur, and enhancing the adhesive force between the interlayer film and a lamination glass member. It is also an object of the present invention to provide a laminated glass prepared with the interlayer film for laminated glass.

### MEANS FOR SOLVING THE PROBLEMS

According to a broad aspect of the present invention, there is provided an interlayer film for laminated glass (hereinafter, also referred to as interlayer film) having a one-layer or two or more-layer structure, the interlayer film for laminated glass containing an ultraviolet absorber represented by the following formula (X), and a metal salt.

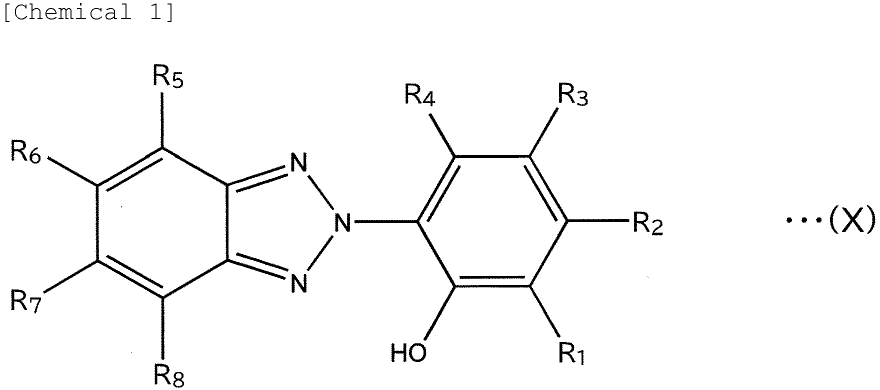

In the above formula (X), R₁ represents any group, R₂ to R₈ each represent a hydrogen atom, an atom other than hydrogen atom, or any group.

In a specific aspect of the interlayer film according to the present invention, the interlayer film includes a layer containing the ultraviolet absorber and the metal salt.

In a specific aspect of the interlayer film according to the present invention, in the above formula (X), R₁ is an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group.

In a specific aspect of the interlayer film according to the present invention, the ultraviolet absorber has a molecular weight of 355 or more.

In a specific aspect of the interlayer film according to the present invention, the ultraviolet absorber includes an ultraviolet absorber represented by the following formula (X11), the following formula (X12) or the following formula (X13).

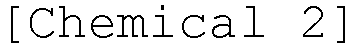

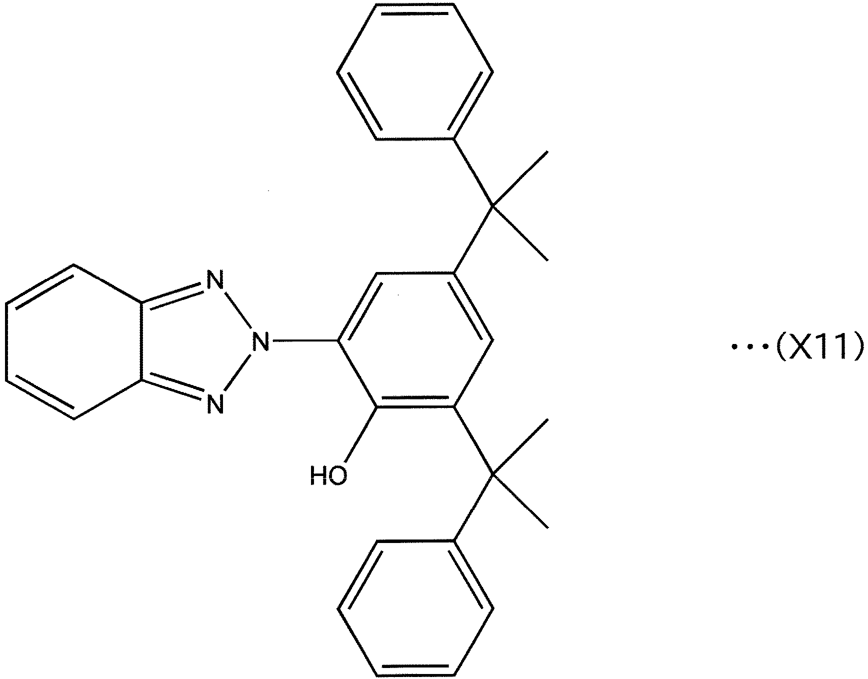

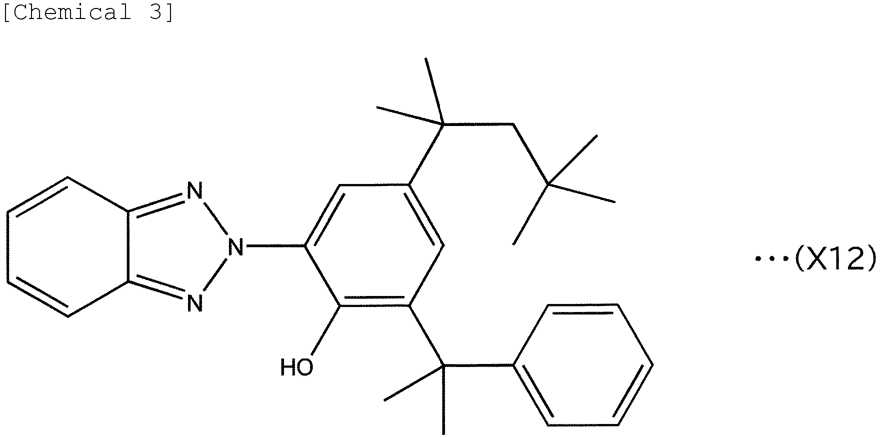

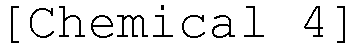

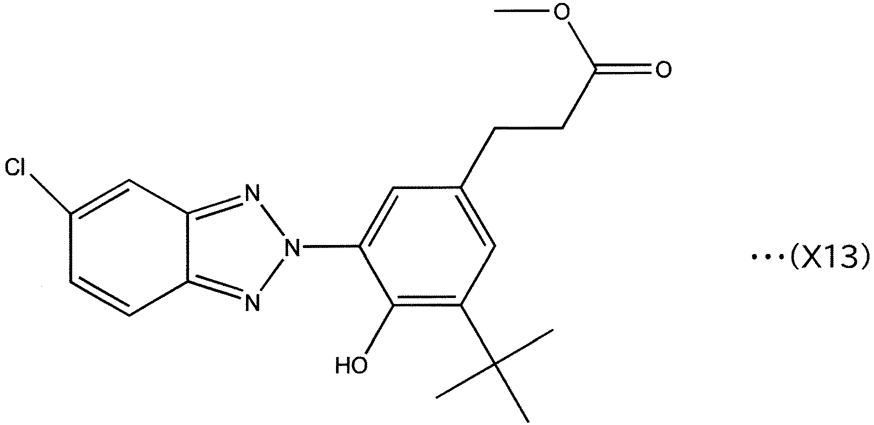

In a specific aspect of the interlayer film according to the present invention, the metal salt includes an alkali metal salt or an alkali earth metal salt.

In a specific aspect of the interlayer film according to the present invention, the metal salt includes a magnesium salt of an organic acid having a branched structure.

In a specific aspect of the interlayer film according to the present invention, the metal salt is a metal salt other than a magnesium salt of an organic acid having a branched structure, and includes a metal salt of an organic acid having 2 or more and 8 or less carbon atoms.

In a specific aspect of the interlayer film according to the present invention, the interlayer film is an interlayer film for laminated glass having a two or more-layer structure, and includes a first layer, and a second layer arranged on a first surface side of the first layer.

In a specific aspect of the interlayer film according to the present invention, the second layer is a surface layer of the interlayer film, and the second layer contains the ultraviolet absorber and the metal salt.

In a specific aspect of the interlayer film according to the present invention, the interlayer film is an interlayer film for laminated glass having a three or more-layer structure, and includes a third layer arranged on a second surface side opposite to the first surface of the first layer.

In a specific aspect of the interlayer film according to the present invention, the third layer is a surface layer of the interlayer film, and the third layer contains the ultraviolet absorber and the metal salt.

In a specific aspect of the interlayer film according to the present invention, the interlayer film includes a layer containing the ultraviolet absorber and the metal salt, and in the layer containing the ultraviolet absorber and the metal salt, a weight ratio of a content of metal contained in the metal salt to a content of the ultraviolet absorber is 4 or more and 50 or less.

In a specific aspect of the interlayer film according to the present invention, the interlayer film has a maximum value of transmittance at a wavelength of 300 nm or more and 350 nm or less of 0.1% or less.

In a specific aspect of the interlayer film according to the present invention, the interlayer film has an ultraviolet transmittance Tuv of 0.5% or less.

In a specific aspect of the interlayer film according to the present invention, the interlayer film has a transmittance at a wavelength of 400 nm of 1.5% or more.

In a specific aspect of the interlayer film according to the present invention, an absolute value of difference between yellow index YI of the interlayer film, and yellow index YI of an interlayer film for comparison having the same layer configuration and thickness as the interlayer film except for not containing a metal salt is 0.1 or less.

According to a broad aspect of the present invention, there is provided a laminated glass including a first lamination glass member, a second lamination glass member, and the above-described interlayer film for laminated glass, the interlayer film for laminated glass being arranged between the first lamination glass member and the second lamination glass member.

### EFFECT OF THE INVENTION

The interlayer film for laminated glass according to the present invention has a one-layer structure or a two or more-layer structure. The interlayer film for laminated glass according to the present invention contains an ultraviolet absorber represented by formula (X), and a metal salt. In the interlayer film for laminated glass according to the present invention, since the above configuration is provided, it is possible to make yellowing less likely to occur, and enhance the adhesive force between the interlayer film and a lamination glass member.

### BRIEF DESCRIPTION OF DRAWINGS

[Fig. 1] Fig. 1 is a sectional view schematically showing an interlayer film for laminated glass in accordance with a first embodiment of the present invention.
[Fig. 2] Fig. 2 is a sectional view schematically showing an interlayer film for laminated glass in accordance with a second embodiment of the present invention.
[Fig. 3] Fig. 3 is a sectional view schematically showing an example of laminated glass prepared with the interlayer film for laminated glass shown in Fig. 1.
[Fig. 4] Fig. 4 is a sectional view schematically showing an example of laminated glass prepared with the interlayer film for laminated glass shown in Fig. 2.

### MODES FOR CARRYING OUT THE INVENTION

Hereinafter, the details of the present invention will be described.

### (Interlayer film for laminated glass)

The interlayer film for laminated glass (in the present specification, sometimes abbreviated as "interlayer film") according to the present invention is used for laminated glass.

The interlayer film according to the present invention has a one-layer structure or a two or more-layer structure. The interlayer film according to the present invention may have a one-layer structure and may have a two or more-layer structure. The interlayer film according to the present invention may have a two-layer structure, may have a two or more-layer structure, may have a three-layer structure, and may have a three or more-layer structure. The interlayer film according to the present invention may include only a first layer. The interlayer film according to the present invention may include, a first layer, and a second layer arranged on a first surface side of the first layer. The interlayer film according to the present invention may include a first layer, a second layer arranged on a first surface side of the first layer, and a third layer arranged on a second surface side opposite to the first surface of the first layer. The interlayer film according to the present invention may be a single-layered interlayer film and may be a multi-layered interlayer film. The structure of the interlayer film according to the present invention may partially vary. For example, the interlayer film according to the present invention may have a part having a one-layer structure, and a part having a multi-layer structure.

The interlayer film according to the present invention contains an ultraviolet absorber represented by the following formula (X) (in the present specification, also abbreviated as "ultraviolet absorber (X)"), and a metal salt. Therefore, the interlayer film according to the present invention contains ultraviolet absorber (X), and a metal salt.

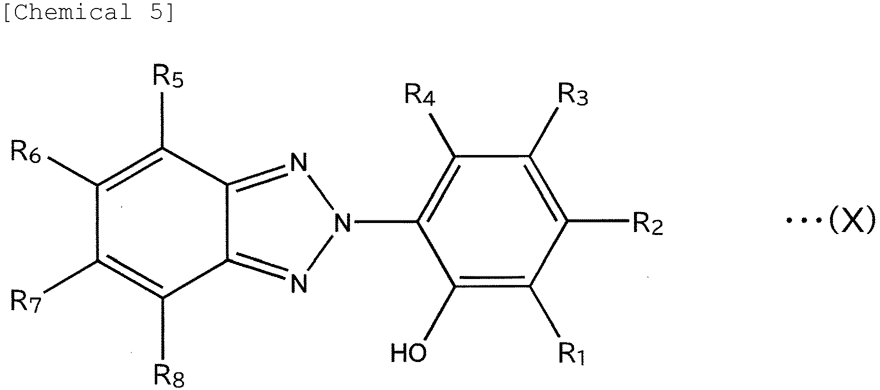

In the above formula (X), R₁ represents any group, and R₂ to R₈ each represent a hydrogen atom, an atom other than hydrogen atom, or any group.

With a conventional combination of an ultraviolet absorber having a benzotriazole skeleton, and a metal salt, yellowing sometimes occur in the interlayer film. Also, as an ultraviolet absorber having a benzotriazole skeleton, an ultraviolet absorber having a skeleton in which a hydroxyl group is directly bonded to a benzene ring (phenol skeleton) is sometimes used. The present inventors found that occurrence of yellowing in the interlayer film is caused by reaction between the hydroxyl group of the ultraviolet absorber having a benzotriazole skeleton and the metal salt. Although the yellowing can be suppressed to some extent by reducing the content of the metal salt, the adhesive force between the interlayer film and a lamination glass member deteriorates.

In contrast to this, in the interlayer film according to the present invention, since ultraviolet absorber (X) having any group at a specific position of the benzotriazole skeleton (position of R₁ in the formula (X) is used, it is possible to reduce the reactivity between the hydroxyl group directly bonded to the benzene ring and the metal salt. Therefore, it is possible to make yellowing less likely to occur in the interlayer film. Further, since the metal salt is used in the interlayer film according to the present invention, it is possible to enhance the adhesive force between the interlayer film and a lamination glass member.

That is, in the interlayer film according to the present invention, it is possible to make yellowing less likely to occur, and enhance the adhesive force between the interlayer film and a lamination glass member although the ultraviolet absorber having a benzotriazole skeleton and the metal salt are contained.

Also, in the interlayer film according to the present invention, it is possible to enhance the adhesive force between layers in the interlayer film when the interlayer film has a two or more-layer structure.

It is preferred that the interlayer film include a layer containing ultraviolet absorber (X) and a metal salt. When the interlayer film is a single-layered interlayer film having a one-layer structure, the interlayer film includes only the first layer containing ultraviolet absorber (X) and a metal salt. When the interlayer film is a multi-layered interlayer film having a two or more-layer structure, it is preferred that the interlayer film include at least one layer containing ultraviolet absorber (X) and a metal salt. When the interlayer film is a multi-layered interlayer film having a two or more-layer structure, it is more preferred that at least one surface layer of the interlayer film be a layer containing ultraviolet absorber (X) and a metal salt, and it is further preferred that two surface layers of the interlayer film be layers containing ultraviolet absorber (X) and a metal salt. When the interlayer film is a multi-layered interlayer film having a two or more-layer structure, it is more preferred that the second layer be a surface layer of the interlayer film, and the second layer be a layer containing ultraviolet absorber (X) and a metal salt. When the interlayer film is a multi-layered interlayer film having a three or more-layer structure, it is more preferred that the third layer be a surface layer of the interlayer film, and the third layer be a layer containing ultraviolet absorber (X) and a metal salt. When the interlayer film is a multi-layered interlayer film having a two or more-layer structure, it is most preferred that all layers of the interlayer film be layers containing ultraviolet absorber (X) and a metal salt.

Hereinafter, specific embodiments of the present invention will be described with reference to the drawings.

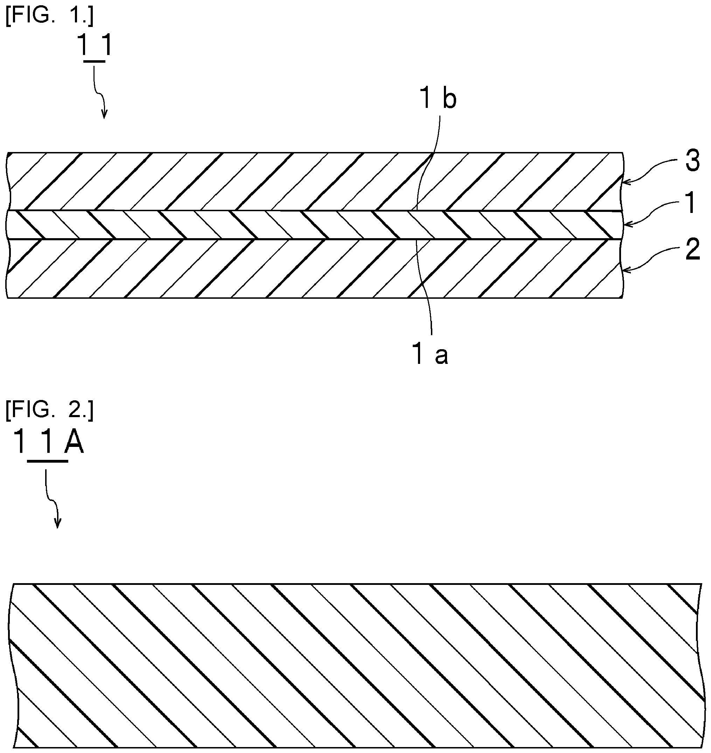

Fig. 1 is a sectional view schematically showing an interlayer film for laminated glass in accordance with a first embodiment of the present invention. In Fig. 1, a section in the thickness direction of an interlayer film 11 is shown.

The interlayer film 11 shown in Fig. 1 is a multi-layered interlayer film having a two or more-layer structure. The interlayer film 11 is used for obtaining laminated glass. The interlayer film 11 is an interlayer film for laminated glass. The interlayer film 11 includes a first layer 1, a second layer 2, and a third layer 3. The second layer 2 is arranged on a first surface 1a of the first layer 1 to be layered thereon. The third layer 3 is arranged on a second surface 1b opposite to the first surface 1a of the first layer 1 to be layered thereon. The first layer 1 is an intermediate layer. Each of the second layer 2 and the third layer 3 is a protective layer and is a surface layer in the present embodiment. The first layer 1 is arranged between the second layer 2 and the third layer 3 to be sandwiched therebetween. Accordingly, the interlayer film 11 has a multilayer structure (second layer 2/first layer 1/third layer 3) in which the second layer 2, the first layer 1, and the third layer 3 are layered in this order.

The first layer 1 contains ultraviolet absorber (X) and a metal salt. The second layer 2 contains ultraviolet absorber (X) and a metal salt. The third layer 3 contains ultraviolet absorber (X) and a metal salt. In the interlayer film 11, each layer provided in the interlayer film 11 contains ultraviolet absorber (X) and a metal salt.

In this connection, other layers may be arranged between the second layer 2 and the first layer 1 and between the first layer 1 and the third layer 3, respectively. Examples of other layers include a layer containing polyethylene terephthalate and the like. It is preferred that the second layer 2 and the first layer 1, and the first layer 1 and the third layer 3 be directly layered, respectively.

Fig. 2 is a sectional view schematically showing an interlayer film for laminated glass in accordance with a second embodiment of the present invention. In Fig. 2, a section in the thickness direction of an interlayer film 11A is shown.

The interlayer film 11A shown in Fig. 2 is a single-layered interlayer film having a one-layer structure. The interlayer film 11A is a first layer. The interlayer film 11A is used for obtaining laminated glass. The interlayer film 11A is an interlayer film for laminated glass. The interlayer film 11A contains ultraviolet absorber (X) and a metal salt.

Hereinafter, the details of the first layer, the second layer and the third layer which constitute the interlayer film according to the present invention, and the details of each ingredient contained in the first layer, the second layer and the third layer will be described.

### <Ultraviolet absorber represented by formula (X) (ultraviolet absorber (X))>

The interlayer film contains ultraviolet absorber (X). The interlayer film includes a layer containing ultraviolet absorber (X). Ultraviolet absorber (X) is an ultraviolet absorber represented by the following formula (X). It is preferred that the first layer contain ultraviolet absorber (X). It is preferred that the second layer contain ultraviolet absorber (X). It is preferred that the third layer contain ultraviolet absorber (X). One kind of ultraviolet absorber (X) may be used alone, and two or more kinds thereof may be used in combination. Ultraviolet absorber (X) contained in the first layer, ultraviolet absorber (X) contained in the second layer, and ultraviolet absorber (X) contained in the third layer may be the same or different from each other.

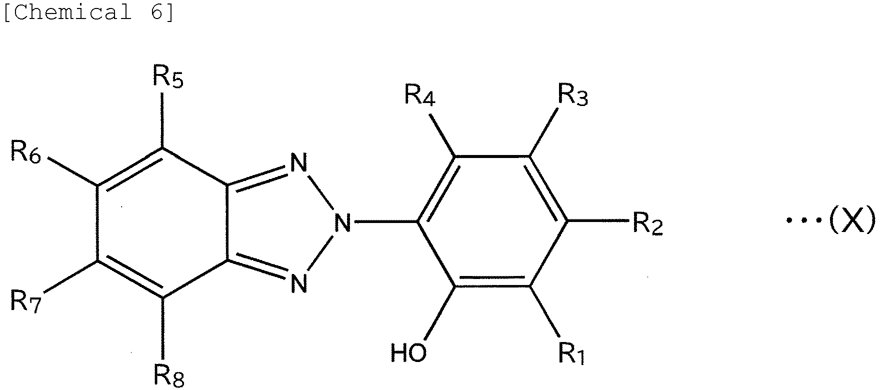

In the above formula (X), R₁ represents any group, and R₂ to R₈ each represent a hydrogen atom, an atom other than hydrogen atom, or any group.

In the above formula (X), R₁ is preferably a group having one or more carbon atoms, more preferably a group having three or more carbon atoms, and is preferably a group having 20 or less carbon atoms, more preferably a group having 10 or less carbon atoms. In this case, it is possible to further reduce the reactivity between the hydroxyl group directly bonded to the benzene ring in ultraviolet absorber (X) and the metal salt, and thus it is possible to make yellowing still less likely to occur.

In the above formula (X), it is preferred that R₁ be an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. In the above formula (X), it is more preferred that R₁ be a propyl group, a group represented by the following formula (R11) or a group represented by the following formula (R12). In this case, it is possible to still further reduce the reactivity between the hydroxyl group directly bonded to the benzene ring in ultraviolet absorber (X) and the metal salt, and thus it is possible to make yellowing still less likely to occur.

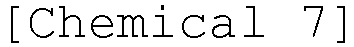

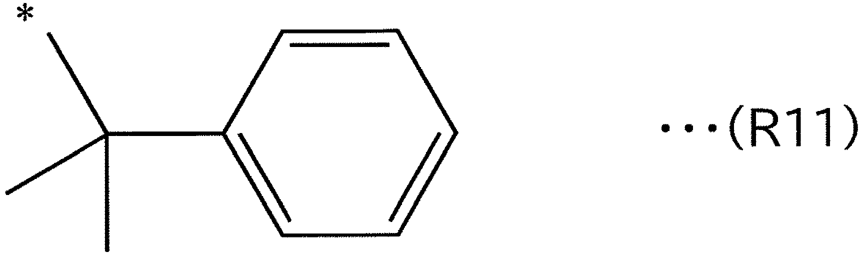

In the above formula (R11), * indicates a bonding position with a carbon atom constituting a benzene ring.

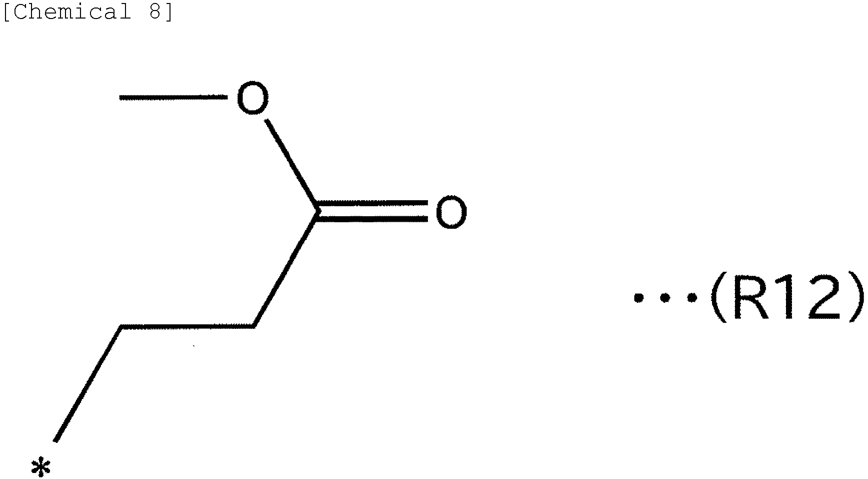

In the above formula (R12), * indicates a bonding position with a carbon atom constituting a benzene ring.

In the above formula (X), it is preferred that R₂ be a hydrogen atom, an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. From the viewpoint of making yellowing still less likely to occur, in the above formula (X), it is preferred that R₂ be an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. From the viewpoint of the accessibility of ultraviolet absorber (X) and suppressibility of yellowing, it is preferred that in the above formula (X), R₂ be a hydrogen atom.

In the above formula (X), R₃ is preferably a group having one or more carbon atoms, more preferably a group having three or more carbon atoms, and is preferably a group having 30 or less carbon atoms, more preferably a group having 20 or less carbon atoms, further preferably a group having 10 or less carbon atoms. In this case, it is possible to make yellowing still less likely to occur while keeping the ultraviolet absorptivity high.

In the above formula (X), it is preferred that R₃ be an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. In the above formula (X), it is more preferred that R₃ be a propyl group, a group represented by the above formula (R11) or a group represented by the above formula (R12). In this case, it is possible to still further reduce the reactivity between the hydroxyl group directly bonded to the benzene ring in ultraviolet absorber (X) and the metal salt, and thus it is possible to make yellowing still less likely to occur.

In the above formula (X), it is preferred that R₄ be a hydrogen atom, an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. From the viewpoint of making yellowing still less likely to occur, in the above formula (X), it is preferred that R₄ be an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. From the viewpoint of the accessibility of ultraviolet absorber (X) and suppressibility of yellowing, it is preferred that in the above formula (X), R₄ be a hydrogen atom.

In the above formula (X), it is preferred that R₅ be a hydrogen atom, an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. From the viewpoint of making yellowing still less likely to occur, in the above formula (X), it is preferred that R₅ be an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. From the viewpoint of the accessibility of ultraviolet absorber (X) and suppressibility of yellowing, it is preferred that in the above formula (X), Rs be a hydrogen atom.

In the above formula (X), it is preferred that R₆ be a hydrogen atom, an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. From the viewpoint of making yellowing still less likely to occur, in the above formula (X), it is preferred that R₆ be an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. From the viewpoint of the accessibility of ultraviolet absorber (X) and suppressibility of yellowing, it is preferred that in the above formula (X), R₆ be a hydrogen atom.

In the above formula (X), it is preferred that R₇ be a hydrogen atom, an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. From the viewpoint of making yellowing still less likely to occur, in the above formula (X), it is preferred that R₇ be an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. From the viewpoint of the accessibility of ultraviolet absorber (X) and suppressibility of yellowing, it is preferred that in the above formula (X), R₇ be a hydrogen atom.

In the above formula (X), it is preferred that R₈ be a hydrogen atom, an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. From the viewpoint of making yellowing still less likely to occur, in the above formula (X), it is preferred that R₈ be an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. From the viewpoint of the accessibility of ultraviolet absorber (X) and suppressibility of yellowing, it is preferred that in the above formula (X), R₈ be a hydrogen atom.

It is preferred that ultraviolet absorber (X) include an ultraviolet absorber represented by the following formula (X1), and it is more preferred that ultraviolet absorber (X) be an ultraviolet absorber represented by the following formula (X1). In this case, it is possible to make yellowing still less likely to occur while keeping the ultraviolet absorptivity high.

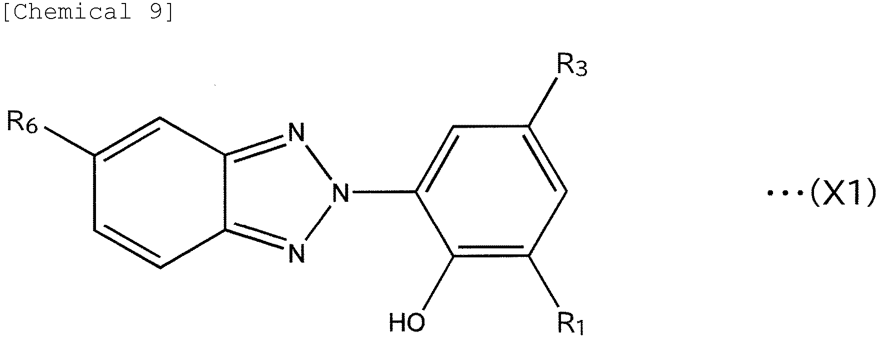

In the above formula (X1), R₁ represents any group, R₃ represents a group having one or more carbon atoms, and R₆ represents a hydrogen atom or a halogen atom.

In the above formula (X1), R₁ is preferably a group having one or more carbon atoms, more preferably a group having three or more carbon atoms, and is preferably a group having 20 or less carbon atoms, more preferably a group having 10 or less carbon atoms. In this case, it is possible to further reduce the reactivity between the hydroxyl group directly bonded to the benzene ring in ultraviolet absorber (X) and the metal salt, and thus it is possible to make yellowing still less likely to occur.

In the above formula (X1), it is preferred that R₁ be an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. In the above formula (X1), it is more preferred that R₁ be a propyl group, a group represented by the above formula (R11) or a group represented by the above formula (R12). In this case, it is possible to still further reduce the reactivity between the hydroxyl group directly bonded to the benzene ring in ultraviolet absorber (X) and the metal salt, and thus it is possible to make yellowing still less likely to occur.

In the above formula (X1), R₃ is preferably a group having one or more carbon atoms, and is preferably a group having 30 or less carbon atoms, more preferably a group having 10 or less carbon atoms. In this case, it is possible to make yellowing still less likely to occur while keeping the ultraviolet absorptivity high.

In the formula (X1), it is preferred that R₆ be a hydrogen atom or a chlorine atom. In this case, it is possible to make yellowing still less likely to occur while keeping the ultraviolet absorptivity high.

It is preferred that ultraviolet absorber (X) include an ultraviolet absorber represented by the following formula (X11), the following formula (X12) or the following formula (X13), and it is more preferred that ultraviolet absorber (X) be an ultraviolet absorber represented by the following formula (X11), the following formula (X12) or the following formula (X13). In this case, it is possible to make yellowing still less likely to occur while keeping the ultraviolet absorptivity high.

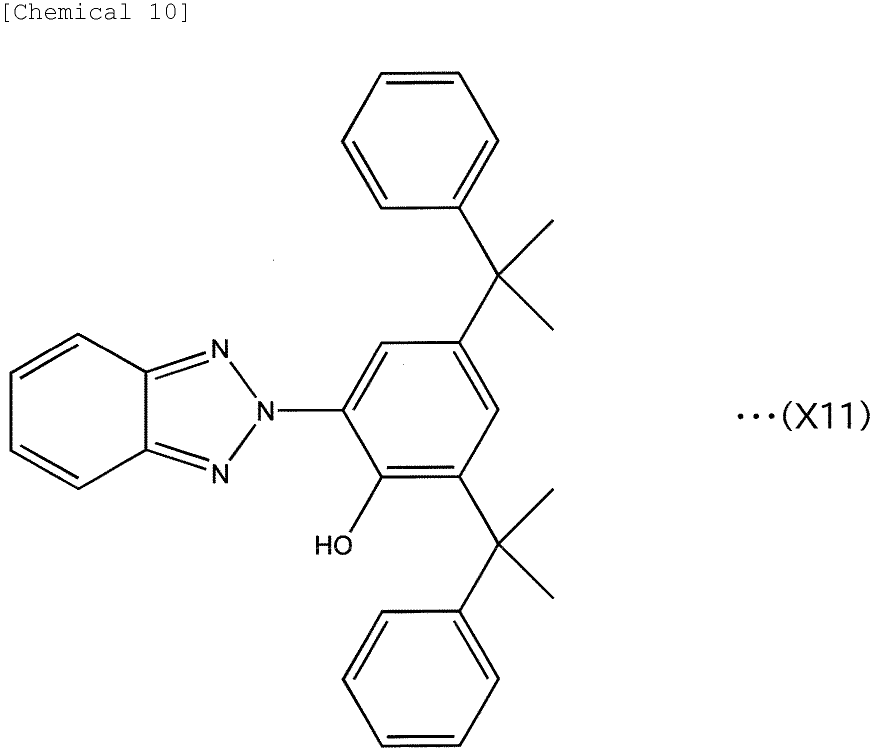

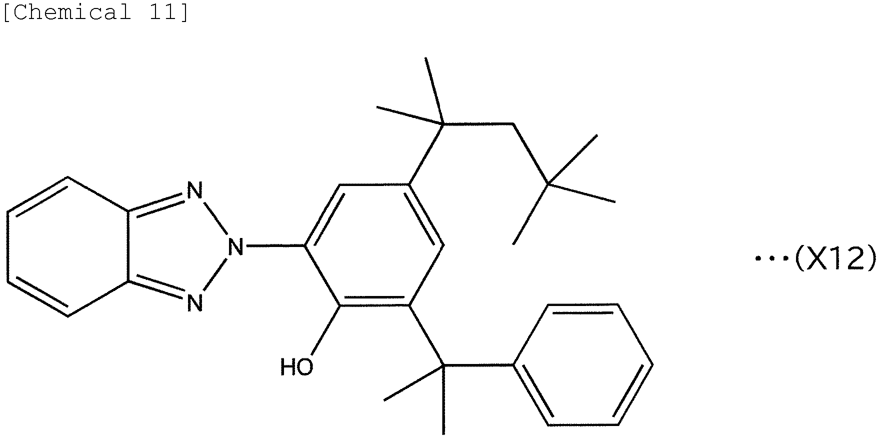

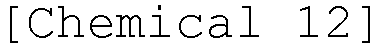

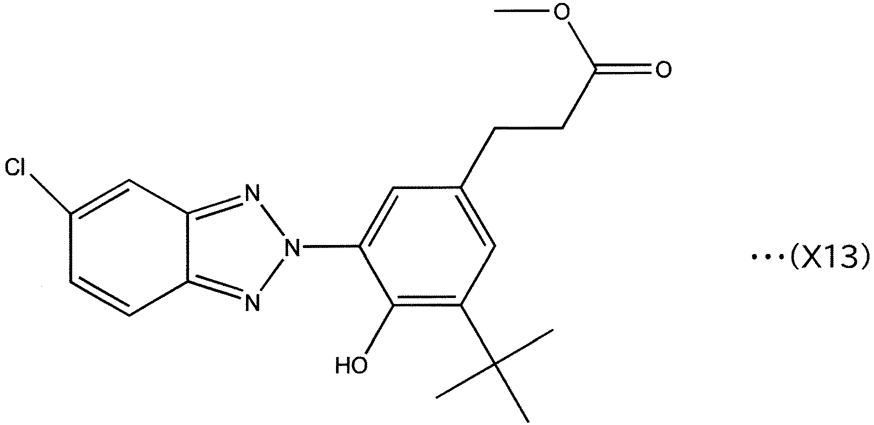

A molecular weight of ultraviolet absorber (X) is preferably 350 or more, more preferably 355 or more, further preferably 380 or more, and is preferably 600 or less, more preferably 500 or less. When the molecular weight of ultraviolet absorber (X) is the above lower limit or more and the above upper limit or less, it is possible to make yellowing still less likely to occur while keeping the ultraviolet absorptivity high.

Examples of commercially available products of ultraviolet absorber (X) include "Tinuvin 234" and "Tinuvin 640" available from BASF Japan Ltd., "RIASORB UV-234" and "RIASORB UV-928" available from Rianlon, "Eversorb 88" and "Eversorb 89" available from Everlight Chemical, "Viosorb234" available from KYODO CHEMICAL CO., LTD., "SONGSORB 2340" and "ONGSORB 9280" available from Songwon, "Eusorb UV-234" available from Eutec, "CHIGUARD 234" and "CHIGUARD 5228" available from Chitec Technology Co., Ltd. and the like.

In 100% by weight of a layer containing ultraviolet absorber (X) (a first layer, a second layer, or a third layer), a content of ultraviolet absorber (X) is preferably 0.1% by weight or more, more preferably 0.2% by weight or more, further preferably 0.3% by weight or more, especially preferably 0.4% by weight or more and is preferably 7% by weight or less, more preferably 6% by weight or less, further preferably 5% by weight or less, especially preferably 4% by weight or less. When the content of ultraviolet absorber (X) is the above lower limit or more, it is possible to further reduce the ultraviolet transmittance Tuv of the interlayer film, and also it is possible to further suppress deterioration in visible light transmittance even after long-term use of the interlayer film and the laminated glass. In particular, by setting the content of ultraviolet absorber (X) to 0.1% by weight or more in 100% by weight of a layer containing ultraviolet absorber (X), it is possible to significantly suppress deterioration in visible light transmittance even after long-term use of the interlayer film and the laminated glass. When the content of ultraviolet absorber (X) is the above upper limit or less, it is possible to further enhance the dispersibility of ultraviolet absorber (X) in the layer containing ultraviolet absorber (X).

In 100% by weight of the interlayer film, the content of ultraviolet absorber (X) is preferably 0.1% by weight or more, more preferably 0.2% by weight or more, further preferably 0.3% by weight or more, especially preferably 0.4% by weight or more and is preferably 7% by weight or less, more preferably 6% by weight or less, further preferably 5% by weight or less, especially preferably 4% by weight or less. When the content of ultraviolet absorber (X) is the above lower limit or more, it is possible to further reduce the ultraviolet transmittance Tuv of the interlayer film, and also it is possible to further suppress deterioration in visible light transmittance even after long-term use of the interlayer film and the laminated glass. In particular, by setting the content of ultraviolet absorber (X) to 0.1% by weight or more in 100% by weight of the interlayer film, it is possible to significantly suppress deterioration in visible light transmittance even after long-term use of the interlayer film and the laminated glass. When the content of ultraviolet absorber (X) is the above upper limit or less, it is possible to further enhance the dispersibility of ultraviolet absorber (X) in the interlayer film.

Relative to 100 parts by weight of the thermoplastic resin in a layer containing ultraviolet absorber (X) (a first layer, a second layer, or a third layer), a content of ultraviolet absorber (X) in the layer containing the ultraviolet absorber (X) is preferably 0.1 parts by weight or more, more preferably 0.2 parts by weight or more, further preferably 0.3 parts by weight or more, and is preferably 3 parts by weight or less, more preferably 2.5 parts by weight or less, further preferably 2 parts by weight or less. When the content of ultraviolet absorber (X) is the above lower limit or more, it is possible to further reduce the ultraviolet transmittance Tuv of the interlayer film, and also it is possible to further suppress deterioration in visible light transmittance even after long-term use of the interlayer film and the laminated glass. When the content of ultraviolet absorber (X) is the above upper limit or less, it is possible to further enhance the dispersibility of ultraviolet absorber (X) in the layer containing ultraviolet absorber (X).

### (Metal salt)

The interlayer film contains a metal salt. The interlayer film includes a layer containing a metal salt. It is preferred that the first layer contain the metal salt. It is preferred that the second layer contain the metal salt. It is preferred that the third layer contain the metal salt. It is preferred that the layer containing ultraviolet absorber (X) contain the metal salt. By the use of the metal salt, controlling the adhesivity between the interlayer film and a lamination glass member such as a glass plate or the adhesivity between layers in the interlayer film is facilitated. One kind of the metal salt may be used alone, and two or more kinds thereof may be used in combination. The metal salt contained in the first layer, the metal salt contained in the second layer, and the metal salt contained in the third layer may be the same or different from each other.

It is preferred that the metal salt include an alkali metal salt or an alkali earth metal salt. In this case, controlling the adhesivity between the interlayer film and a lamination glass member or the adhesivity between layers in the interlayer film is facilitated.

The alkali earth metal means six metals of Be, Mg, Ca, Sr, Ba, and Ra.

It is preferred that the metal salt contain at least one kind of metal selected from the group consisting of Li, Na, K, Rb, Cs, Mg, Ca, Sr and Ba. The metal salt contained in the interlayer film preferably contains at least one metal of K and Mg, and more preferably contains Mg. Since the metal salt contains Mg, it is possible to improve the collision safety at a low moisture content.

It is preferred that the metal salt include a magnesium salt of an organic acid having a branched structure (P). In this case, controlling the adhesivity between the interlayer film and a lamination glass member such as a glass plate or the adhesivity between layers in the interlayer film is facilitated.

It is preferred that the magnesium salt of an organic acid having a branched structure (P) be a magnesium salt of a carboxylic acid having a branched structure. In this case, controlling the adhesivity between the interlayer film and a lamination glass member such as a glass plate or the adhesivity between layers in the interlayer film is further facilitated.

It is preferred that the metal salt be a metal salt other than a magnesium salt of an organic acid having a branched structure and include a metal salt of an organic acid having 2 or more and 8 or less carbon atoms (Q). The metal salt of an organic acid having 2 or more and 8 or less carbon atoms (Q) is different from the magnesium salt of an organic acid having a branched structure (P). Also in this case, controlling the adhesivity between the interlayer film and a lamination glass member such as a glass plate or the adhesivity between layers in the interlayer film is further facilitated.

It is preferred the metal salt of an organic acid having 2 or more and 8 or less carbon atoms (Q) be a magnesium salt or a potassium salt of an organic acid having 2 or more and 8 or less carbon atoms. In this case, controlling the adhesivity between the interlayer film and a lamination glass member such as a glass plate or the adhesivity between layers in the interlayer film is still further facilitated.

It is also preferred that the metal salt include the magnesium salt of an organic acid having a branched structure (P), and the metal salt of an organic acid having 2 or more and 8 or less carbon atoms (Q).

As the metal salt, an alkali metal salt of an organic acid having 2 to 16 carbon atoms, and an alkali earth metal salt of an organic acid having 2 to 16 carbon atoms can be used. The metal salt may include a magnesium carboxylate having 2 to 16 carbon atoms, or a potassium carboxylate having 2 to 16 carbon atoms.

Examples of the magnesium carboxylate having 2 to 16 carbon atoms and the potassium carboxylate having 2 to 16 carbon atoms include magnesium acetate, potassium acetate, magnesium propionate, potassium propionate, magnesium 2-ethylbutyrate, potassium 2-ethylbutanoate, magnesium 2-ethylhexanoate, potassium 2-ethylhexanoate, and the like.

In a layer containing ultraviolet absorber (X) and a metal salt, a weight ratio of the content of the metal contained in the metal salt to the content of the ultraviolet absorber (X) (content of metal contained in metal salt/content of ultraviolet absorber (X)) is preferably 0.1 or more, more preferably 1.5 or more, further preferably 4 or more, especially preferably 5 or more, and is preferably 50 or less, more preferably 35 or less. When the weight ratio (content of metal contained in metal salt/content of ultraviolet absorber (X)) is the above lower limit or more and the above upper limit or less, it is possible to exert the effect of the present invention more effectively.

The total of the contents of Mg and K in an interlayer film containing the metal salt or a layer containing the metal salt (a first layer, a second layer, a third layer, or a layer containing ultraviolet absorber (X)) is preferably 5 ppm or more, more preferably 10 ppm or more, further preferably 20 ppm or more, and is preferably 300 ppm or less, more preferably 250 ppm or less, further preferably 200 ppm or less. When the total of the contents of Mg and K is the above lower limit or more and the above upper limit or less, the adhesivity between the interlayer film and a lamination glass member (e.g., glass plate) or the adhesivity between layers in the interlayer film can be further well controlled.

The content of Mg in an interlayer film containing the metal salt or a layer containing the metal salt (a first layer, a second layer, a third layer, or a layer containing ultraviolet absorber (X)) is preferably 5 ppm or more, more preferably 10 ppm or more, further preferably 20 ppm or more, and is preferably 300 ppm or less, more preferably 250 ppm or less, further preferably 200 ppm or less.

The contents of Mg and K, and the content of Mg can be determined from a blending amount of the metal salt contained in each layer, or can be determined by measurement using an ICP optical emission spectrometer.

### (Thermoplastic resin)

It is preferred that the interlayer film contain a thermoplastic resin (hereinafter, sometimes described as a thermoplastic resin (0)). It is preferred that the interlayer film contain a polyvinyl acetal resin (hereinafter, sometimes described as a polyvinyl acetal resin (0)) as the thermoplastic resin (0). It is preferred that the first layer contain a thermoplastic resin (hereinafter, sometimes described as a thermoplastic resin (1)). It is preferred that the first layer contain a polyvinyl acetal resin (hereinafter, sometimes described as a polyvinyl acetal resin (1)) as the thermoplastic resin (1). It is preferred that the second layer contain a thermoplastic resin (hereinafter, sometimes described as a thermoplastic resin (2)). It is preferred that the second layer contain a polyvinyl acetal resin (hereinafter, sometimes described as a polyvinyl acetal resin (2)) as the thermoplastic resin (2). It is preferred that the third layer contain a thermoplastic resin (hereinafter, sometimes described as a thermoplastic resin (3)). It is preferred that the third layer contain a polyvinyl acetal resin (hereinafter, sometimes described as a polyvinyl acetal resin (3)) as the thermoplastic resin (3). It is preferred that the layer containing ultraviolet absorber (X) contain a thermoplastic resin (hereinafter, sometimes described as a thermoplastic resin (4)). It is preferred that the layer containing ultraviolet absorber (X) contain a polyvinyl acetal resin (hereinafter, sometimes described as a polyvinyl acetal resin (4)) as the thermoplastic resin (4). The thermoplastic resin (1), the thermoplastic resin (2), the thermoplastic resin (3), and the thermoplastic resin (4) may be the same or different from one another. For still higher sound insulating property, it is preferred that the thermoplastic resin (1) be different from the thermoplastic resin (2) and the thermoplastic resin (3). Each of the polyvinyl acetal resin (1), the polyvinyl acetal resin (2), the polyvinyl acetal resin (3), and the polyvinyl acetal resin (4) may be the same or different from one another. For still higher sound insulating property, it is preferred that the polyvinyl acetal resin (1) be different from the polyvinyl acetal resin (2) and the polyvinyl acetal resin (3). One kind of each of the thermoplastic resin (0), the thermoplastic resin (1), the thermoplastic resin (2), the thermoplastic resin (3), and the thermoplastic resin (4) may be used alone and two or more kinds thereof may be used in combination. One kind of each of the polyvinyl acetal resin (0), the polyvinyl acetal resin (1), the polyvinyl acetal resin (2), the polyvinyl acetal resin (3), and the polyvinyl acetal resin (4) may be used alone and two or more kinds thereof may be used in combination.

Examples of the thermoplastic resin include a polyvinyl acetal resin, an ethylene-vinyl acetate copolymer resin, an ethylene-acrylic acid copolymer resin, a polyurethane resin, a (meth)acrylic resin, a polyolefin resin, an ionomer resin, a polyvinyl alcohol resin, and the like. Thermoplastic resins other than these may be used.

For example, the polyvinyl acetal resin can be produced by acetalizing polyvinyl alcohol (PVA) with an aldehyde. It is preferred that the polyvinyl acetal resin be an acetalized product of polyvinyl alcohol. The polyvinyl alcohol can be obtained, for example, by saponifying polyvinyl acetate. The saponification degree of the polyvinyl alcohol generally lies within the range of 70% by mole to 99.9% by mole.

The average polymerization degree of the polyvinyl alcohol (PVA) is preferably 200 or more, more preferably 500 or more, even more preferably 1500 or more, further preferably 1600 or more, especially preferably 2600 or more, most preferably 2700 or more and is preferably 5000 or less, more preferably 4000 or less, further preferably 3500 or less. When the average polymerization degree is the above lower limit or more, the penetration resistance of the laminated glass is further enhanced. When the average polymerization degree is the aforementioned upper limit or less, formation of an interlayer film is facilitated.

The average polymerization degree of the polyvinyl alcohol is determined by a method in accordance with JIS K6726 "Testing methods for polyvinyl alcohol".

The number of carbon atoms of the acetal group contained in the polyvinyl acetal resin is not particularly limited. The aldehyde used at the time of producing the polyvinyl acetal resin is not particularly limited. The number of carbon atoms of the acetal group in the polyvinyl acetal resin is preferably 3 to 5, more preferably 3 or 4. When the number of carbon atoms of the acetal group in the polyvinyl acetal resin is 3 or more, the glass transition temperature of the interlayer film is sufficiently lowered. The number of carbon atoms of the acetal group in the polyvinyl acetal resin may be 4 or 5.

The aldehyde is not particularly limited. In general, an aldehyde having 1 to 10 carbon atoms is suitably used. Examples of the aldehyde having 1 to 10 carbon atoms include propionaldehyde, n-butyraldehyde, isobutyraldehyde, n-valeraldehyde, 2-ethylbutyraldehyde, n-hexylaldehyde, n-octylaldehyde, n-nonylaldehyde, n-decylaldehyde, formaldehyde, acetaldehyde, benzaldehyde, and the like. The aldehyde is preferably propionaldehyde, n-butyraldehyde, isobutyraldehyde, n-hexylaldehyde, or n-valeraldehyde, more preferably propionaldehyde, n-butyraldehyde or isobutyraldehyde, and further preferably n-butyraldehyde. One kind of the aldehyde may be used alone, and two or more kinds thereof may be used in combination.

The content of the hydroxyl group (the amount of hydroxyl groups) of the polyvinyl acetal resin (0) is preferably 15% by mole or more, more preferably 18% by mole or more and is preferably 40% by mole or less, more preferably 35% by mole or less. When the content of the hydroxyl group is the above lower limit or more, the adhesive force of the interlayer film is further enhanced. Moreover, when the content of the hydroxyl group is the aforementioned upper limit or less, the flexibility of the interlayer film is enhanced and the handling of the interlayer film is facilitated.

A content of the hydroxyl group (the amount of hydroxyl groups) of the polyvinyl acetal resin (1) is preferably 17% by mole or more, more preferably 20% by mole or more, further preferably 22% by mole or more and is preferably 28% by mole or less, more preferably 27% by mole or less, further preferably 25% by mole or less, especially preferably 24% by mole or less. When the content of the hydroxyl group is the above lower limit or more, the mechanical strength of the interlayer film is further enhanced. In particular, when the content of the hydroxyl group of the polyvinyl acetal resin (1) is 20% by mole or more, the resin is high in reaction efficiency and is excellent in productivity, and moreover, when being 28% by mole or less, the sound insulating property of laminated glass is further enhanced. Moreover, when the content of the hydroxyl group is the aforementioned upper limit or less, the flexibility of the interlayer film is enhanced and the handling of the interlayer film is facilitated.

A preferred range of the content of the hydroxyl group of the polyvinyl acetal resin (4) when the layer containing ultraviolet absorber (X) is not a surface layer of the interlayer film, is the same as a preferred range of the content of the hydroxyl group of the polyvinyl acetal resin (1).

The content of the hydroxyl group (hydroxyl group amount) of the polyvinyl acetal resin (2) and the polyvinyl acetal resin (3) is preferably 25% by mole or more, more preferably 28% by mole or more, more preferably 30% by mole or more, still more preferably 31.5% by mole or more, further preferably 32% by mole or more, especially preferably 33% by mole or more. The content of the hydroxyl group (hydroxyl group amount) of the polyvinyl acetal resin (2) and the polyvinyl acetal resin (3) is preferably 38% by mole or less, more preferably 37% by mole or less, further preferably 36.5% by mole or less, especially preferably 36% by mole or less. When the content of the hydroxyl group is the above lower limit or more, the adhesive force of the interlayer film is further enhanced. Moreover, when the content of the hydroxyl group is the aforementioned upper limit or less, the flexibility of the interlayer film is enhanced and the handling of the interlayer film is facilitated.

A preferred range of the content of the hydroxyl group of the polyvinyl acetal resin (4) when the layer containing ultraviolet absorber (X) is a surface layer of the interlayer film, is the same as a preferred range of the content of the hydroxyl group of the polyvinyl acetal resin (2) and the polyvinyl acetal resin (3).

From the viewpoint of further enhancing the sound insulating property, it is preferred that the content of the hydroxyl group of the polyvinyl acetal resin (1) be lower than the content of the hydroxyl group of the polyvinyl acetal resin (2). From the viewpoint of further enhancing the sound insulating property, it is preferred that the content of the hydroxyl group of the polyvinyl acetal resin (1) be lower than the content of the hydroxyl group of the polyvinyl acetal resin (3). An absolute value of difference between the content of the hydroxyl group of the polyvinyl acetal resin (1) and the content of the hydroxyl group of the polyvinyl acetal resin (2) is defined as absolute value A, and an absolute value of difference between the content of the hydroxyl group of the polyvinyl acetal resin (1) and the content of the hydroxyl group of the polyvinyl acetal resin (3) is defined as absolute value B. From the viewpoint of further enhancing the sound insulating property, each of the absolute value A and the absolute value B is preferably 1% by mole or more, more preferably 5% by mole or more, further preferably 9% by mole or more, especially preferably 10% by mole or more, most preferably 12% by mole or more. Each of the absolute value A and the absolute value B is preferably 20% by mole or less.

When the layer containing ultraviolet absorber (X) is not a surface layer of the interlayer film, from the viewpoint of further enhancing the sound insulating property, it is preferred that the content of the hydroxyl group of the polyvinyl acetal resin (4) be lower than the content of the hydroxyl group of the polyvinyl acetal resin (2). When the layer containing ultraviolet absorber (X) is not a surface layer of the interlayer film, from the viewpoint of further enhancing the sound insulating property, it is preferred that the content of the hydroxyl group of the polyvinyl acetal resin (4) be lower than the content of the hydroxyl group of the polyvinyl acetal resin (3). An absolute value of difference between the content of the hydroxyl group of the polyvinyl acetal resin (4) and the content of the hydroxyl group of the polyvinyl acetal resin (2) is defined as absolute value C, and an absolute value of difference between the content of the hydroxyl group of the polyvinyl acetal resin (4) and the content of the hydroxyl group of the polyvinyl acetal resin (3) is defined as absolute value D. From the viewpoint of further enhancing the sound insulating property, each of the absolute value C and the absolute value D is preferably 1% by mole or more, more preferably 5% by mole or more, further preferably 9% by mole or more, especially preferably 10% by mole or more, most preferably 12% by mole or more. Each of the absolute value C and the absolute value D is preferably 20% by mole or less.

When the layer containing ultraviolet absorber (X) is a surface layer of the interlayer film, from the viewpoint of further enhancing the sound insulating property, it is preferred that the content of the hydroxyl group of the polyvinyl acetal resin (1) be lower than the content of the hydroxyl group of the polyvinyl acetal resin (4). When the layer containing ultraviolet absorber (X) is a surface layer of the interlayer film, from the viewpoint of still further enhancing the sound insulating property, the absolute value of difference between the content of the hydroxyl group of the polyvinyl acetal resin (1) and the content of the hydroxyl group of the polyvinyl acetal resin (4) is preferably 1% by mole or more, more preferably 5% by mole or more, further preferably 9% by mole or more, especially preferably 10% by mole or more, most preferably 12% by mole or more. An absolute value of difference between the content of the hydroxyl group of the polyvinyl acetal resin (1) and the content of the hydroxyl group of the polyvinyl acetal resin (4) is preferably 20% by mole or less.

The content of the hydroxyl group of the polyvinyl acetal resin is a mole fraction, represented in percentage, obtained by dividing the amount of ethylene groups to which the hydroxyl group is bonded by the total amount of ethylene groups in the main chain. For example, the amount of ethylene groups to which the hydroxyl group is bonded can be measured in accordance with JIS K6728 "Testing methods for polyvinyl butyral".

The acetylation degree (the amount of acetyl groups) of the polyvinyl acetal resin (0) is preferably 0.1% by mole or more, more preferably 0.3% by mole or more, further preferably 0.5% by mole or more and is preferably 30% by mole or less, more preferably 25% by mole or less, and further preferably 20% by mole or less. When the acetylation degree is the above lower limit or more, the compatibility between the polyvinyl acetal resin and a plasticizer is enhanced. When the acetylation degree is the aforementioned upper limit or less, the moisture resistance of the interlayer film and laminated glass is enhanced.

The acetylation degree (the amount of acetyl groups) of the polyvinyl acetal resin (1) is preferably 0.01% by mole or more, more preferably 0.1% by mole or more, even more preferably 7% by mole or more, further preferably 9% by mole or more and is preferably 30% by mole or less, more preferably 25% by mole or less, further preferably 24% by mole or less, especially preferably 20% by mole or less. When the acetylation degree is the above lower limit or more, the compatibility between the polyvinyl acetal resin and a plasticizer is enhanced. When the acetylation degree is the aforementioned upper limit or less, the moisture resistance of the interlayer film and laminated glass is enhanced. In particular, when the acetylation degree of the polyvinyl acetal resin (1) is 0.1% by mole or more and is 25% by mole or less, the resulting laminated glass is excellent in penetration resistance.

A preferred range of the acetylation degree of the polyvinyl acetal resin (4) when the layer containing ultraviolet absorber (X) is not a surface layer of the interlayer film, is the same as a preferred range of the acetylation degree of the polyvinyl acetal resin (1).

The acetylation degree (acetyl group amount) of each of the polyvinyl acetal resin (2) and the polyvinyl acetal resin (3) is preferably 0.01% by mole or more, more preferably 0.5% by mole or more, and is preferably 10% by mole or less, more preferably 2% by mole or less. When the acetylation degree is the above lower limit or more, the compatibility between the polyvinyl acetal resin and a plasticizer is enhanced. When the acetylation degree is the aforementioned upper limit or less, the moisture resistance of the interlayer film and laminated glass is enhanced.

A preferred range of the acetylation degree of the polyvinyl acetal resin (4) when the layer containing ultraviolet absorber (X) is a surface layer of the interlayer film, is the same as a preferred range of the acetylation degree of the polyvinyl acetal resin (2) and the polyvinyl acetal resin (3).

The acetylation degree is a mole fraction, represented in percentage, obtained by dividing the amount of ethylene groups to which the acetyl group is bonded by the total amount of ethylene groups in the main chain. For example, the amount of ethylene groups to which the acetyl group is bonded can be determined in accordance with JIS K6728 "Testing methods for polyvinyl butyral".

The acetalization degree of the polyvinyl acetal resin (0) (the butyralization degree in the case of a polyvinyl butyral resin) is preferably 60% by mole or more, more preferably 63% by mole or more and is preferably 85% by mole or less, more preferably 75% by mole or less, further preferably 70% by mole or less. When the acetalization degree is the above lower limit or more, the compatibility between the polyvinyl acetal resin and a plasticizer is enhanced. When the acetalization degree is the aforementioned upper limit or less, the reaction time required for producing the polyvinyl acetal resin is shortened.

The acetalization degree of the polyvinyl acetal resin (1) (the butyralization degree in the case of a polyvinyl butyral resin) is preferably 47% by mole or more, more preferably 60% by mole or more, and is preferably 85% by mole or less, more preferably 80% by mole or less, further preferably 75% by mole or less. When the acetalization degree is the above lower limit or more, the compatibility between the polyvinyl acetal resin and a plasticizer is enhanced. When the acetalization degree is the aforementioned upper limit or less, the reaction time required for producing the polyvinyl acetal resin is shortened.

A preferred range of the acetalization degree of the polyvinyl acetal resin (4) when the layer containing ultraviolet absorber (X) is not a surface layer of the interlayer film, is the same as a preferred range of the acetalization degree of the polyvinyl acetal resin (1).

The acetalization degree of the polyvinyl acetal resin (2) and the polyvinyl acetal resin (3) (the butyralization degree in the case of a polyvinyl butyral resin) is preferably 55% by mole or more and more preferably 60% by mole or more and is preferably 75% by mole or less and more preferably 71% by mole or less. When the acetalization degree is the above lower limit or more, the compatibility between the polyvinyl acetal resin and a plasticizer is enhanced. When the acetalization degree is the aforementioned upper limit or less, the reaction time required for producing the polyvinyl acetal resin is shortened.

A preferred range of the acetalization degree of the polyvinyl acetal resin (4) when the layer containing ultraviolet absorber (X) is a surface layer of the interlayer film, is the same as a preferred range of the acetalization degree of the polyvinyl acetal resin (2) and the polyvinyl acetal resin (3).

The acetalization degree is determined in the following manner. From the total amount of the ethylene group in the main chain, the amount of the ethylene group to which the hydroxyl group is bonded and the amount of the ethylene group to which the acetyl group is bonded are subtracted. The obtained value is divided by the total amount of the ethylene group in the main chain to obtain a mole fraction. The mole fraction represented in percentage is the acetalization degree.

In this connection, it is preferred that the content of the hydroxyl group (the amount of hydroxyl groups), the acetalization degree (the butyralization degree) and the acetylation degree be calculated from the results determined by a method in accordance with JIS K6728 "Testing methods for polyvinyl butyral". In this context, a method in accordance with ASTM D1396-92 may be used. When the polyvinyl acetal resin is a polyvinyl butyral resin, the content of the hydroxyl group (the amount of hydroxyl groups), the acetalization degree (the butyralization degree) and the acetylation degree can be calculated from the results measured by a method in accordance with JIS K6728 "Testing methods for polyvinyl butyral".

In 100% by weight of the thermoplastic resin contained in the interlayer film, the content of the polyvinyl acetal resin is preferably 10% by weight or more, more preferably 30% by weight or more, still more preferably 50% by weight or more, further preferably 70% by weight or more, especially preferably 80% by weight or more, most preferably 90% by weight or more. In 100% by weight of the thermoplastic resin contained in the interlayer film, the content of the polyvinyl acetal resin is preferably 100% by weight or less. It is preferred that the main ingredient (50% by weight or more) of the thermoplastic resin of the interlayer film be a polyvinyl acetal resin.

In 100% by weight of the thermoplastic resin contained in the first layer, the content of the polyvinyl acetal resin is preferably 10% by weight or more, more preferably 30% by weight or more, still more preferably 50% by weight or more, further preferably 70% by weight or more, especially preferably 80% by weight or more, most preferably 90% by weight or more. In 100% by weight of the thermoplastic resin contained in the first layer, the content of the polyvinyl acetal resin is preferably 100% by weight or less. It is preferred that the main ingredient (50% by weight or more) of the thermoplastic resin of the first layer be a polyvinyl acetal resin.

In 100% by weight of the thermoplastic resin contained in the second layer, the content of the polyvinyl acetal resin is preferably 10% by weight or more, more preferably 30% by weight or more, still more preferably 50% by weight or more, further preferably 70% by weight or more, especially preferably 80% by weight or more, most preferably 90% by weight or more. In 100% by weight of the thermoplastic resin contained in the second layer, the content of the polyvinyl acetal resin is preferably 100% by weight or less. It is preferred that the main ingredient (50% by weight or more) of the thermoplastic resin of the second layer be a polyvinyl acetal resin.

In 100% by weight of the thermoplastic resin contained in the third layer, the content of the polyvinyl acetal resin is preferably 10% by weight or more, more preferably 30% by weight or more, still more preferably 50% by weight or more, further preferably 70% by weight or more, especially preferably 80% by weight or more, most preferably 90% by weight or more. In 100% by weight of the thermoplastic resin contained in the third layer, the content of the polyvinyl acetal resin is preferably 100% by weight or less. It is preferred that the main ingredient (50% by weight or more) of the thermoplastic resin of the third layer be a polyvinyl acetal resin.

In 100% by weight of the thermoplastic resin contained in the layer containing ultraviolet absorber (X), the content of the polyvinyl acetal resin is preferably 10% by weight or more, more preferably 30% by weight or more, still more preferably 50% by weight or more, further preferably 70% by weight or more, especially preferably 80% by weight or more, most preferably 90% by weight or more. In 100% by weight of the thermoplastic resin contained in the layer containing ultraviolet absorber (X), the content of the polyvinyl acetal resin is preferably 100% by weight or less. It is preferred that the main ingredient (50% by weight or more) of the thermoplastic resin in the layer containing ultraviolet absorber (X) be a polyvinyl acetal resin.

### (Plasticizer)

From the viewpoint of further enhancing the adhesive force of an interlayer film, it is preferred that the interlayer film according to the present invention contain a plasticizer (hereinafter, sometimes described as a plasticizer (0)). It is preferred that the first layer contain a plasticizer (hereinafter, sometimes described as a plasticizer (1)). It is preferred that the second layer contain a plasticizer (hereinafter, sometimes described as a plasticizer (2)). It is preferred that the third layer contain a plasticizer (hereinafter, sometimes described as a plasticizer (3)). It is preferred that the layer containing ultraviolet absorber (X) contain a plasticizer (hereinafter, sometimes described as a plasticizer (4)). When the thermoplastic resin contained in the interlayer film is a polyvinyl acetal resin, it is especially preferred that the interlayer film (each layer) contain a plasticizer. It is preferred that a layer containing a polyvinyl acetal resin contain a plasticizer.

The plasticizer is not particularly limited. As the plasticizer, a conventionally known plasticizer can be used. One kind of the plasticizer may be used alone and two or more kinds thereof may be used in combination.

Examples of the plasticizer include organic ester plasticizers such as a monobasic organic acid ester and a polybasic organic acid ester, organic phosphate plasticizers and organic phosphite plasticizers, and the like. It is preferred that the plasticizer be an organic ester plasticizer. It is preferred that the plasticizer be a liquid plasticizer.

Examples of the monobasic organic acid ester include a glycol ester obtained by the reaction of a glycol with a monobasic organic acid, and the like. Examples of the glycol include triethylene glycol, tetraethylene glycol, tripropylene glycol, and the like. Examples of the monobasic organic acid include butyric acid, isobutyric acid, caproic acid, 2-ethylbutyric acid, heptanoic acid, n-octylic acid, 2-ethylhexanoic acid, n-nonylic acid, decylic acid, benzoic acid and the like.

Examples of the polybasic organic acid ester include an ester compound of a polybasic organic acid and an alcohol having a linear or branched structure of 4 to 8 carbon atoms, and the like. Examples of the polybasic organic acid include adipic acid, sebacic acid, azelaic acid, and the like.

Examples of the organic ester plasticizer include triethylene glycol di-2-ethylpropanoate, triethylene glycol di-2-ethylbutyrate, triethylene glycol di-2-ethylhexanoate, triethylene glycol dicaprylate, triethylene glycol di-n-octanoate, triethylene glycol di-n-heptanoate, tetraethylene glycol di-n-heptanoate, dibutyl sebacate, dioctyl azelate, dibutyl carbitol adipate, ethylene glycol di-2-ethylbutyrate, 1,3-propylene glycol di-2-ethylbutyrate, 1,4-butylene glycol di-2-ethylbutyrate, diethylene glycol di-2-ethylbutyrate, diethylene glycol di-2-ethylhexanoate, dipropylene glycol di-2-ethylbutyrate, triethylene glycol di-2-ethylpentanoate, tetraethylene glycol di-2-ethylbutyrate, diethylene glycol dicaprylate, diethylene glycol dibenzoate, dipropylene glycol dibenzoate, dihexyl adipate, dioctyl adipate, hexyl cyclohexyl adipate, a mixture of heptyl adipate and nonyl adipate, diisononyl adipate, diisodecyl adipate, heptyl nonyl adipate, dibutyl sebacate, oil-modified sebacic alkyds, a mixture of a phosphoric acid ester and an adipic acid ester, and the like. As the organic ester plasticizer, other organic ester plasticizer than those recited above may be used. As the adipic acid ester, adipic acid esters other than the aforementioned adipic acid esters may be used.

Examples of the organic phosphate plasticizer include tributoxyethyl phosphate, isodecyl phenyl phosphate, triisopropyl phosphate, and the like.

It is preferred that the plasticizer be a diester plasticizer represented by the following formula (1).

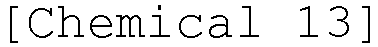

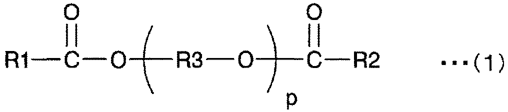

In the formula (1), R1 and R2 each represent an organic group having 2 to 10 carbon atoms, R3 represents an ethylene group, an isopropylene group, or an n-propylene group, and p represents an integer of 3 to 10. R1 and R2 in the formula (1) each are preferably an organic group having 5 to 10 carbon atoms, and more preferably an organic group having 6 to 10 carbon atoms.

It is preferred that the plasticizer include triethylene glycol di-2-ethylhexanoate (3GO), triethylene glycol di-2-ethylbutyrate (3GH) or triethylene glycol di-2-ethylpropanoate. It is more preferred that the plasticizer include triethylene glycol di-2-ethylhexanoate (3GO) or triethylene glycol di-2-ethylbutyrate (3GH), and it is further preferred that the plasticizer include triethylene glycol di-2-ethylhexanoate (3GO).

In the interlayer film, the content of the plasticizer (0) per 100 parts by weight of the thermoplastic resin (0) is defined as content (0). The content (0) is preferably 5 parts by weight or more, more preferably 25 parts by weight or more, further preferably 30 parts by weight or more, and is preferably 100 parts by weight or less, more preferably 60 parts by weight or less, further preferably 50 parts by weight or less. When the content (0) is the above lower limit or more, the penetration resistance of the laminated glass is further enhanced. When the content (0) is the above upper limit or less, the transparency of the interlayer film is further enhanced.

In the first layer, the content of the plasticizer (1) relative to 100 parts by weight of the thermoplastic resin (1) is referred to as content (1). The content (1) is preferably 50 parts by weight or more, more preferably 55 parts by weight or more, further preferably 60 parts by weight or more. The content (1) is preferably 100 parts by weight or less, more preferably 90 parts by weight or less, further preferably 85 parts by weight or less, especially preferably 80 parts by weight or less. When the content (1) is the above lower limit or more, the flexibility of the interlayer film is enhanced and the handling of the interlayer film is facilitated. When the content (1) is the above upper limit or less, the penetration resistance of laminated glass is further enhanced.

When the layer containing ultraviolet absorber (X) is not a surface layer of the interlayer film, a preferred range of the content of the plasticizer (4) relative to 100 parts by weight of the thermoplastic resin (4) (hereinafter, sometimes described as content (4)) is the same with a preferred range of the content (1) in the layer containing ultraviolet absorber (X).

In the second layer, the content of the plasticizer (2) per 100 parts by weight of the thermoplastic resin (2) is referred to as a content (2). In the third layer, the content of the plasticizer (3) relative to 100 parts by weight of the thermoplastic resin (3) is referred to as content (3). Each of the content (2) and the content (3) is preferably 5 parts by weight or more, more preferably 10 parts by weight or more, still more preferably 15 parts by weight or more, further preferably 20 parts by weight or more, especially preferably 24 parts by weight or more, and most preferably 25 parts by weight or more. Each of the content (2) and the content (3) is preferably 45 parts by weight or less, more preferably 40 parts by weight or less, further preferably 35 parts by weight or less, especially preferably 32 parts by weight or less, and most preferably 30 parts by weight or less. When the content (2) and the content (3) are the above lower limit or more, the flexibility of the interlayer film is enhanced and the handling of the interlayer film is facilitated. When the content (2) and the content (3) are the above upper limit or less, the penetration resistance of the laminated glass is further enhanced.

When the layer containing ultraviolet absorber (X) is a surface layer of the interlayer film, a preferred range of the content of the plasticizer (4) relative to 100 parts by weight of the thermoplastic resin (4) (hereinafter, sometimes described as content (4)) is the same with a preferred range of the content (2) and the content (3) in the layer containing ultraviolet absorber (X) .

For the purpose of enhancing the sound insulating property of the laminated glass, it is preferred that the content (1) be larger than the content (2) and it is preferred that the content (1) be larger than the content (3).

When the layer containing ultraviolet absorber (X) is not a surface layer of the interlayer film, for the purpose of enhancing the sound insulating property of laminated glass, it is preferred that the content (4) be larger than the content (2) and it is preferred that the content (4) be larger than the content (3).

When the layer containing ultraviolet absorber (X) is a surface layer of the interlayer film, for the purpose of enhancing the sound insulating property of laminated glass, it is preferred that the content (1) be larger than the content (4).

From the viewpoint of further enhancing the sound insulating property of the laminated glass, each of the absolute value of difference between the content (2) and the content (1) and the absolute value of difference between the content (3) and the content (1) is preferably 10 parts by weight or more, more preferably 15 parts by weight or more, and further preferably 20 parts by weight or more. Each of the absolute value of difference between the content (2) and the content (1) and the absolute value of difference between the content (3) and the content (1) is preferably 80 parts by weight or less, more preferably 75 parts by weight or less, further preferably 70 parts by weight or less.

When the layer containing ultraviolet absorber (X) is not a surface layer of the interlayer film, from the viewpoint of further enhancing the sound insulating property of laminated glass, each of the absolute value of difference between the content (2) and the content (4) and the absolute value of difference between the content (3) and the content (4) is preferably 10 parts by weight or more, more preferably 15 parts by weight or more, and further preferably 20 parts by weight or more. Each of the absolute value of difference between the content (2) and the content (4) and the absolute value of difference between the content (3) and the content (4) is preferably 80 parts by weight or less, more preferably 75 parts by weight or less, further preferably 70 parts by weight or less.

When the layer containing ultraviolet absorber (X) is a surface layer of the interlayer film, from the viewpoint of further enhancing the sound insulating property of laminated glass, the absolute value of difference between the content (4) and the content (1) is preferably 10 parts by weight or more, more preferably 15 parts by weight or more, further preferably 20 parts by weight or more. The absolute value of difference between the content (4) and the content (1) is preferably 80 parts by weight or less, more preferably 75 parts by weight or less, further preferably 70 parts by weight or less.

### (Heat shielding substance)

It is preferred that the interlayer film contain a heat shielding substance. It is preferred that the first layer contain a heat shielding substance. It is preferred that the second layer contain a heat shielding substance. It is preferred that the third layer contain a heat shielding substance. It is preferred that the layer containing ultraviolet absorber (X) contain a heat shielding substance. One kind of the heat shielding substance may be used alone, and two or more kinds thereof may be used in combination.

It is preferred that the heat shielding substance contain at least one kind of Ingredient X among a phthalocyanine compound, a naphthalocyanine compound, and an anthracyanine compound or contain heat shielding particles. In this case, the heat shielding substance may contain both of the Ingredient X and the heat shielding particles.

### Ingredient X:

It is preferred that the interlayer film contain at least one kind of Ingredient X among a phthalocyanine compound, a naphthalocyanine compound, and an anthracyanine compound. It is preferred that the first layer contain the Ingredient X. It is preferred that the second layer contain the Ingredient X. It is preferred that the third layer contain the Ingredient X. It is preferred that the layer containing ultraviolet absorber (X) contain the Ingredient X. The Ingredient X is a heat shielding substance. One kind of the Ingredient X may be used alone and two or more kinds thereof may be used in combination.

The Ingredient X is not particularly limited. As the Ingredient X, conventionally known phthalocyanine compound, naphthalocyanine compound and anthracyanine compound can be used.

Examples of the Ingredient X include phthalocyanine, a derivative of phthalocyanine, naphthalocyanine, a derivative of naphthalocyanine, anthracyanine, and a derivative of anthracyanine, and the like. It is preferred that each of the phthalocyanine compound and the derivative of phthalocyanine have a phthalocyanine skeleton. It is preferred that each of the naphthalocyanine compound and the derivative of naphthalocyanine have a naphthalocyanine skeleton. It is preferred that each of the anthracyanine compound and the derivative of anthracyanine have an anthracyanine skeleton.

With regard to the interlayer film and laminated glass, from the viewpoint of further enhancing the heat shielding properties thereof, it is preferred that the Ingredient X be at least one kind selected from the group consisting of phthalocyanine, a derivative of phthalocyanine, naphthalocyanine and a derivative of naphthalocyanine, and it is more preferred that the Ingredient X be at least one kind among phthalocyanine and a derivative of phthalocyanine.

From the viewpoints of effectively enhancing the heat shielding property and maintaining the visible light transmittance at a higher level over a long period of time, it is preferred that the Ingredient X contain vanadium atoms or copper atoms. It is preferred that the Ingredient X contain vanadium atoms and it is also preferred that the Ingredient X contain copper atoms. It is more preferred that the Ingredient X be at least one kind among phthalocyanine containing vanadium atoms or copper atoms and a derivative of phthalocyanine containing vanadium atoms or copper atoms. From the viewpoint of still further enhancing the heat shielding property of the interlayer film and the laminated glass, it is preferred that the Ingredient X have a structural unit in which an oxygen atom is bonded to a vanadium atom.

In 100% by weight of the interlayer film or in 100% by weight of a layer containing the Ingredient X (a first layer, a second layer, a third layer, or a layer containing ultraviolet absorber (X)), the content of the Ingredient X is preferably 0.001% by weight or more, more preferably 0.005% by weight or more, further preferably 0.01% by weight or more, especially preferably 0.02% by weight or more. In 100% by weight of the interlayer film or in 100% by weight of a layer containing the Ingredient X (a first layer, a second layer, a third layer, or a layer containing ultraviolet absorber (X)), the content of the Ingredient X is preferably 0.2% by weight or less, more preferably 0.1% by weight or less, further preferably 0.05% by weight or less, especially preferably 0.04% by weight or less. When the content of the Ingredient X is the above lower limit or more and the above upper limit or less, the heat shielding property is sufficiently enhanced and the visible light transmittance is sufficiently enhanced. For example, it is possible to make the visible light transmittance 70% or more.

### Heat shielding particles:

It is preferred that the interlayer film contain heat shielding particles. It is preferred that the first layer contain the heat shielding particles. It is preferred that the second layer contain the heat shielding particles. It is preferred that the third layer contain the heat shielding particles. It is preferred that the layer containing ultraviolet absorber (X) contain the heat shielding particles. The heat shielding particles are a heat shielding substance. By the use of heat shielding particles, infrared rays (heat rays) can be effectively cut off. One kind of the heat shielding particles may be used alone, and two or more kinds thereof may be used in combination.

From the viewpoint of further enhancing the heat shielding properties of laminated glass, it is more preferred that the heat shielding particles be metal oxide particles. It is preferred that the heat shielding particles be particles of a metal oxide (metal oxide particles).

The energy amount of an infrared ray with a wavelength of 780 nm or longer which is longer than that of visible light is small as compared with an ultraviolet ray. However, the thermal action of infrared rays is large, and when infrared rays are absorbed into a substance, heat is released from the substance. Accordingly, infrared rays are generally called heat rays. By the use of the heat shielding particles, infrared rays (heat rays) can be effectively cut off. In this connection, the heat shielding particle means a particle capable of absorbing infrared rays.

Specific examples of the heat shielding particles include metal oxide particles such as aluminum-doped tin oxide particles, indium-doped tin oxide particles, antimony-doped tin oxide particles (ATO particles), gallium-doped zinc oxide particles (GZO particles), indium-doped zinc oxide particles (IZO particles), aluminum-doped zinc oxide particles (AZO particles), niobium-doped titanium oxide particles, sodium-doped tungsten oxide particles, cesium-doped tungsten oxide particles, thallium-doped tungsten oxide particles, rubidium-doped tungsten oxide particles, tin-doped indium oxide particles (ITO particles), tin-doped zinc oxide particles and silicon-doped zinc oxide particles, lanthanum hexaboride (LaB₆) particles, and the like. Heat shielding particles other than these may be used. Since the heat ray shielding function is high, preferred are metal oxide particles, more preferred are ATO particles, GZO particles, IZO particles, ITO particles or tungsten oxide particles, and especially preferred are ITO particles or tungsten oxide particles. In particular, since the heat ray shielding function is high and the particles are readily available, preferred are tin-doped indium oxide particles (ITO particles), and also preferred are tungsten oxide particles.

With regard to the interlayer film and laminated glass, from the viewpoint of further enhancing the heat shielding properties thereof, it is preferred that the tungsten oxide particles be metal-doped tungsten oxide particles. Examples of the "tungsten oxide particles" include metal-doped tungsten oxide particles. Specifically, examples of the metal-doped tungsten oxide particles include sodium-doped tungsten oxide particles, cesium-doped tungsten oxide particles, thallium-doped tungsten oxide particles, rubidium-doped tungsten oxide particles, and the like.

With regard to the interlayer film and laminated glass, from the viewpoint of further enhancing the heat shielding properties thereof, cesium-doped tungsten oxide particles are especially preferred. From the viewpoint of still further enhancing the heat shielding property of the interlayer film and the laminated glass, it is preferred that the cesium-doped tungsten oxide particles be tungsten oxide particles represented by the formula: Cs_{0.33}WO₃.

The average particle diameter of the heat shielding particles is preferably 0.01 um or more, more preferably 0.02 µm or more, and is preferably 0.1 um or less, more preferably 0.05 µm or less. When the average particle diameter is the above lower limit or more, the heat ray shielding property is sufficiently enhanced. When the average particle diameter is the above upper limit or less, the dispersibility of heat shielding particles is enhanced.

The "average particle diameter" refers to the volume average particle diameter. The average particle diameter can be measured using a particle size distribution measuring apparatus ("UPA-EX150" available from NIKKISO CO., LTD.), or the like.

In 100% by weight of the interlayer film or in 100% by weight of a layer containing the heat shielding particles (a first layer, a second layer, a third layer, or a layer containing ultraviolet absorber (X)), the content of the heat shielding particles (in particular, the content of tungsten oxide particles) is preferably 0.01% by weight or more, more preferably 0.1% by weight or more, further preferably 1% by weight or more, especially preferably 1.5% by weight or more. In 100% by weight of the interlayer film or in 100% by weight of a layer containing the heat shielding particles (a first layer, a second layer, a third layer, or a layer containing ultraviolet absorber (X)), the content of the heat shielding particles (in particular, the content of tungsten oxide particles) is preferably 6% by weight or less, more preferably 5.5% by weight or less, further preferably 4% by weight or less, especially preferably 3.5% by weight or less, most preferably 3% by weight or less. When the content of the heat shielding particles is the above lower limit or more and the above upper limit or less, the heat shielding property is sufficiently enhanced and the visible light transmittance is sufficiently enhanced.

### (Oxidation inhibitor)

It is preferred that the interlayer film contain an oxidation inhibitor. It is preferred that the first layer contain an oxidation inhibitor. It is preferred that the second layer contain an oxidation inhibitor. It is preferred that the third layer contain an oxidation inhibitor. It is preferred that the layer containing ultraviolet absorber (X) contain an oxidation inhibitor. One kind of the oxidation inhibitor may be used alone, and two or more kinds thereof may be used in combination.

Examples of the oxidation inhibitor include a phenol-based oxidation inhibitor, a sulfur-based oxidation inhibitor, a phosphorus-based oxidation inhibitor, and the like. The phenol-based oxidation inhibitor is an oxidation inhibitor having a phenol skeleton. The sulfur-based oxidation inhibitor is an oxidation inhibitor containing a sulfur atom. The phosphorus-based oxidation inhibitor is an oxidation inhibitor containing a phosphorus atom.

It is preferred that the oxidation inhibitor be a phenol-based oxidation inhibitor or a phosphorus-based oxidation inhibitor.

Examples of the phenol-based oxidation inhibitor include 2,6-di-t-butyl-p-cresol (BHT), butyl hydroxyanisole (BHA), 2,6-di-t-butyl-4-ethylphenol, stearyl β-(3,5-di-t-butyl-4-hydroxyphenyl)propionate, 2,2'-methylenebis-(4-methyl-6-butylphenol), 2,2'-methylenebis-(4-ethyl-6-t-butylphenol), 4,4'-butylidene-bis-(3-methyl-6-t-butylphenol), 1,1,3-tris-(2-methyl-hydroxy-5-t-butylphenyl)butane, tetrakis[methylene-3-(3',5'-butyl-4-hydroxyphenyl)propionate]methane, 1,3,3-tris-(2-methyl-4-hydroxy-5-t-butylphenol)butane, 1,3,5-trimethyl-2,4,6-tris(3,5-di-t-butyl-4-hydroxybenzyl)benzene, bis(3,3'-t-butylphenol)butyric acid glycol ester, and bis(3-t-butyl-4-hydroxy-5-methylbenzenepropanoic acid)ethylenebis(oxyethylene), and the like. One kind or two or more kinds among these oxidation inhibitors are suitably used.

Examples of the phosphorus-based oxidation inhibitor include tridecyl phosphite, tris(tridecyl) phosphite, triphenyl phosphite, trinonylphenyl phosphite, bis(tridecyl)pentaerithritol diphosphite, bis(decyl)pentaerithritol diphosphite, tris(2,4-dit-butylphenyl) phosphite, bis(2,4-di-t-butyl-6-methylphenyl)ethyl ester phosphorous acid, 2,2'-methylenebis(4,6-di-t-butyl-1-phenyloxy)(2-ethylhexyloxy)phosphorus, and the like. One kind or two or more kinds among these oxidation inhibitors are suitably used.

Examples of a commercial product of the oxidation inhibitor include "IRGANOX 245" available from BASF Japan Ltd., "IRGAFOS 168" available from BASF Japan Ltd., "IRGAFOS 38" available from BASF Japan Ltd., "Sumilizer BHT" available from Sumitomo Chemical Co., Ltd., "H-BHT" available from Sakai Chemical Industry Co., Ltd., "IRGANOX 1010" available from BASF Japan Ltd., and the like.

With regard to the interlayer film and laminated glass, in order to maintain high visible light transmittance thereof over a long period of time, the content of the oxidation inhibitor is preferably 0.03% by weight or more, more preferably 0.1% by weight or more in 100% by weight of the interlayer film or in 100% by weight of the layer containing the oxidation inhibitor (a first layer, a second layer, a third layer, or a layer containing ultraviolet absorber (X)). Moreover, since an effect commensurate with the addition of an oxidation inhibitor is not attained, it is preferred that the content of the oxidation inhibitor be 2% by weight or less in 100% by weight of the interlayer film or in 100% by weight of a layer containing the oxidation inhibitor.

### (Other ingredients)

Each of the interlayer film, the first layer, the second layer, the third layer and the layer containing ultraviolet absorber (X) may contain other ingredients than the above-described ingredients as necessary. Examples of other ingredients include an ultraviolet absorber other than ultraviolet absorber (X), a coloring agent (pigment and dye and the like), a coupling agent, a dispersant, a surfactant, a fire retardant, an antistatic agent, an adhesion adjusting agent other than metal salt, a moisture-proofing agent, a fluorescent brightener, and an infrared absorber, and the like. One kind of these other ingredients may be used alone, and two or more kinds thereof may be used in combination.

### (Other details of interlayer film for laminated glass)

The maximum value of transmittance at a wavelength of 300 nm or more and 350 nm or less of the interlayer film is preferably 0.1% or less, more preferably 0.09% or less, further preferably 0.08% or less. When the maximum value of transmittance is the above upper limit or less, the visible light transmittance becomes still less likely to deteriorate even after long-term use of the interlayer film and the laminated glass. The maximum value of transmittance at a wavelength of 300 nm or more and 350 nm or less of the interlayer film may be 0% or more.

Transmittance at a wavelength of 400 nm of the interlayer film is preferably 1.5% or more, more preferably 3% or more, further preferably 5% or more. When the transmittance is the above lower limit or more, it is possible to further enhance the visible light transmittance.

Transmittance at a wavelength of 300 nm or more and 350 nm or less and transmittance at a wavelength of 400 nm of the interlayer film can be measured in the following manner. The interlayer film is arranged between two sheets of 2.5-mm-thick clear glass conforming to JIS R3202:1996 to obtain a laminated glass A. Transmittance at a wavelength of 300 nm or more and 350 nm or less and transmittance at a wavelength of 400 nm of the obtained laminated glass A are measured. Transmittance at a wavelength of 300 nm or more and 350 nm or less and transmittance at a wavelength of 400 nm in the laminated glass A are respectively defined as transmittance at a wavelength of 300 nm or more and 350 nm or less and transmittance at a wavelength of 400 nm in the interlayer film. The transmittance can be measured using a spectrophotometer ("U-4150" available from Hitachi High-Tech Science Corporation) in accordance with JIS R3211:1998.

Ultraviolet transmittance Tuv of the interlayer film is preferably 0.5% or less, more preferably 0.3% or less, further preferably 0.1% or less. When the ultraviolet transmittance Tuv is the above upper limit or less, the visible light transmittance becomes still less likely to deteriorate even after long-term use of the interlayer film and the laminated glass. Ultraviolet transmittance Tuv of the interlayer film may be 0% or more.

Ultraviolet transmittance Tuv of the interlayer film can be measured in the following manner. The interlayer film is arranged between two sheets of 2.5-mm-thick clear glass conforming to JIS R3202:1996 to obtain a laminated glass A. Transmittance at a wavelength of 300 nm or more and 400 nm or less of the obtained laminated glass A is measured. A value calculated from transmittance at a wavelength of 300 nm or more and 400 nm or less of the laminated glass A in a method conforming to ISO9050 is defined as ultraviolet transmittance Tuv of the interlayer film. The ultraviolet transmittance Tuv can be measured using a spectrophotometer ("U-4150" available from Hitachi High-Tech Science Corporation) in accordance with JIS R3211:1998.

An absolute value of difference between yellow index YI of the interlayer film, and yellow index YI of an interlayer film for comparison having the same layer configuration and thickness as the interlayer film except for not containing a metal salt (absolute value of ΔYI) is determined. The absolute value of difference (absolute value of ΔYI) is an index for reactivity between the ultraviolet absorber and the metal salt. The absolute value of difference (absolute value of ΔYI) is preferably 0.1 or less, more preferably 0.08 or less, further preferably 0.05 or less. When the absolute value of difference (absolute value of ΔYI) is the above upper limit or less, the color of the interlayer film is less likely to change by variation in the adding amount of the metal salt, and the adhesive force can be easily adjusted.

Yellow index YI of the interlayer film is a yellow index calculated from total light transmittance. Yellow index YI in the interlayer film can be measured in the following manner. The interlayer film is arranged between two sheets of 2.5-mm-thick clear glass conforming to JIS R3202:1996 to obtain a laminated glass A. Total light transmittance of the obtained laminated glass A is measured. From total light transmittance of the laminated glass A, yellow index YI of the laminated glass A is calculated in accordance with JIS K7373. Yellow index YI of the laminated glass A is defined as yellow index YI of the interlayer film. Also yellow index YI of the interlayer film for comparison is determined in the same manner.

The total light transmittance of the laminated glass A is measured in the following manner.

The laminated glass A is placed parallel with the normal line of the light axis on the optical path between the light source and the integrating sphere at a position where the laminated glass is in contact with the integrating sphere so that only the transmitted light is received by the integrating sphere with a spectrophotometer. The total light transmittance means a visible light transmittance calculated from the spectral transmittance measured in this condition. The total light transmittance can be measured using a spectrophotometer ("U-4150" available from Hitachi High-Tech Science Corporation).

The method for preparing the laminated glass A is not particularly limited. One example of the method for preparing the laminated glass A is shown below. The laminated glass A is prepared for measuring transmittance at a wavelength of 300 nm or more and 350 nm or less, transmittance at a wavelength of 400 nm, ultraviolet transmittance Tuv, and yellow index YI of the interlayer film.

The interlayer film is sandwiched between two sheets of 2.5-mm-thick clear glass conforming to JIS R3202:1996 to obtain a laminate. The obtained laminate is put into a rubber bag and the inside thereof is degassed for 20 minutes with a degree of vacuum of 2.6 kPa, after which the laminate in the degassed condition is transferred into an oven, and vacuum-pressed by retention at 90°C for 30 minutes, and thus the laminate is preliminarily press-bonded. The preliminarily press-bonded laminate is subjected to press-bonding for 20 minutes under conditions of 135°C and a pressure of 1.2 MPa in an autoclave to obtain the laminated glass A.

In preparation of a laminated glass product using the interlayer film according to the present invention, 2.5-mm-thick clear glass conforming to JIS R3202:1996 may be used, and clear glass other than the 2.5-mm-thick clear glass conforming to JIS R3202:1996 may be used, and a lamination glass member other than clear glass may be used.

The interlayer film has one end and the other end being at an opposite side of the one end. The one end and the other end are both end parts facing each other in the interlayer film.

The interlayer film may be an interlayer film in which the thickness of the one end and the thickness of the other end are the same, or may be an interlayer film in which the thickness of the other end is larger than the thickness of the one end. The interlayer film may be an interlayer film having a uniform thickness, or may be an interlayer film having varying thickness. The sectional shape of the interlayer film may be a rectangular shape or may be a wedge-like shape.

The maximum thickness of the interlayer film is preferably 0.1 mm or more, more preferably 0.25 mm or more, further preferably 0.5 mm or more, especially preferably 0.8 mm or more, and is preferably 3.8 mm or less, more preferably 2.0 mm or less, further preferably 1.5 mm or less.

From the viewpoint of the practical aspect and the viewpoint of sufficiently enhancing the adhesive strength and the penetration resistance, the maximum thickness of a surface layer of the interlayer film is preferably 0.001 mm or more, more preferably 0.2 mm or more, further preferably 0.3 mm or more, and is preferably 1.0 mm or less, more preferably 0.8 mm or less.

From the viewpoint of the practical aspect and the viewpoint of sufficiently enhancing the penetration resistance, the maximum thickness of a layer (intermediate layer) arranged between two surface layers is preferably 0.001 mm or more, more preferably 0.1 mm or more, further preferably 0.2 mm or more and is preferably 0.8 mm or less, more preferably 0.6 mm or less, further preferably 0.3 mm or less.

The distance between one end and the other end of the interlayer film is preferably 3.0 m or less, more preferably 2.0 m or less, especially preferably 1.5 m or less, and is preferably 0.5 m or more, more preferably 0.8 m or more, especially preferably 1.0 m or more.

The interlayer film may be wound to be formed into a roll body of the interlayer film. The roll body may include a winding core and an interlayer film wound on the outer periphery of the winding core.

The method for producing the interlayer film is not particularly limited. In the case of a single-layered interlayer film, examples of the production method of the interlayer film include a method of extruding a resin composition with an extruder. One exemplary method for forming the interlayer film in the case of a multi-layered interlayer film includes separately forming layers by using resin compositions for forming respective layers, and then layering the obtained layers. Further, as a method for producing the interlayer film, a method of layering layers by coextruding resin compositions for forming respective layers using an extruder can be recited. A production method of extrusion-molding is preferred because the method is suitable for continuous production.

For the reason of excellent production efficiency of the interlayer film, it is preferred that the second layer and the third layer contain the same polyvinyl acetal resin. For the reason of excellent production efficiency of the interlayer film, it is more preferred that the second layer and the third layer contain the same polyvinyl acetal resin and the same plasticizer. For the reason of excellent production efficiency of the interlayer film, it is further preferred that the second layer and the third layer be formed of the same resin composition.

It is preferred that the interlayer film have protrusions and recesses on at least one surface of the surfaces of both sides. It is more preferred that the interlayer film have protrusions and recesses on surfaces of both sides. Examples of the method for forming the protrusions and recesses include, but are not particularly limited to, a lip emboss method (melt fracture method), an emboss roll method, a calender roll method, and a profile extrusion method, and the like.

### (Laminated glass)

A laminated glass according to the present invention includes a first lamination glass member, a second lamination glass member and the interlayer film described above. In the laminated glass according to the present invention, the interlayer film is arranged between the first lamination glass member and the second lamination glass member.

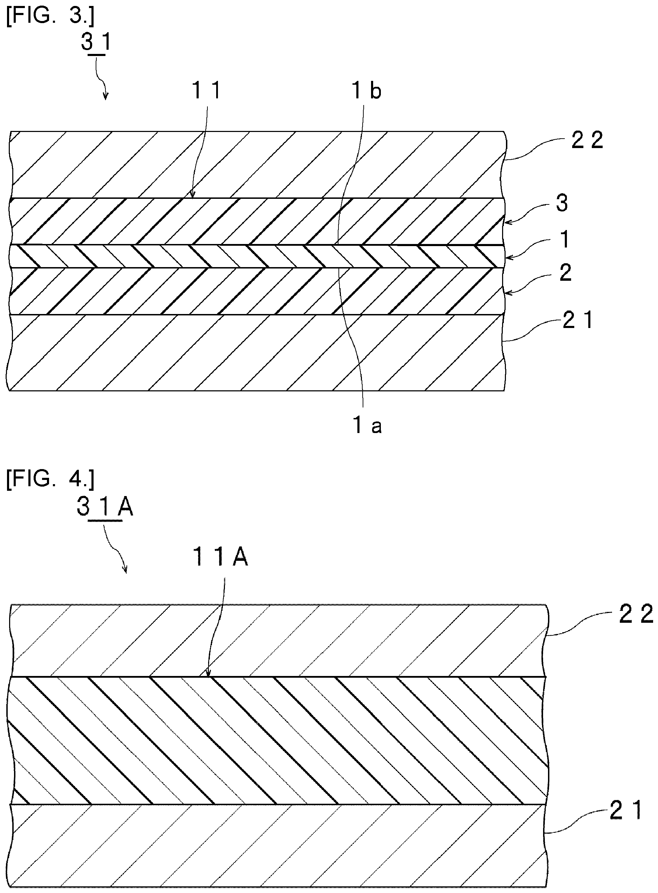

Fig. 3 is a sectional view schematically showing an example of laminated glass prepared with the interlayer film for laminated glass shown in Fig. 1.

A laminated glass 31 shown in Fig. 3 includes a first lamination glass member 21, a second lamination glass member 22 and the interlayer film 11. The interlayer film 11 is arranged between the first lamination glass member 21 and the second lamination glass member 22 to be sandwiched therebetween.

The first lamination glass member 21 is layered on a first surface of the interlayer film 11. The second lamination glass member 22 is layered on a second surface opposite to the first surface of the interlayer film 11. The first lamination glass member 21 is layered on an outer surface of the second layer 2. The second lamination glass member 22 is layered on an outer surface of the third layer 3.

Fig. 4 is a sectional view schematically showing an example of laminated glass prepared with the interlayer film for laminated glass shown in Fig. 2.

A laminated glass 31A shown in Fig. 4 includes the first lamination glass member 21, the second lamination glass member 22 and the interlayer film 11A. The interlayer film 11A is arranged between the first lamination glass member 21 and the second lamination glass member 22 to be sandwiched therebetween.

The first lamination glass member 21 is layered on a first surface of the interlayer film 11A. The second lamination glass member 22 is layered on a second surface opposite to the first surface of the interlayer film 11A.

The laminated glass may be a head-up display. When the laminated glass is a head-up display, the laminated glass has a display region of the head-up display. The display region is a region capable of favorably displaying information.

A head-up display system can be obtained by using the aforementioned head-up display. The head-up display system includes the laminated glass, and a light source device for irradiating the laminated glass with light for image display. The light source device can be attached, for example, to a dashboard in a vehicle. By irradiating the display region of the laminated glass with light from the light source device, it is possible to achieve image display.

It is preferred that the first lamination glass member be the first glass plate. It is preferred that the second lamination glass member be the second glass plate.

Examples of the first and second lamination glass members include a glass plate, a PET (polyethylene terephthalate) film, and the like. As the laminated glass, laminated glass in which an interlayer film is sandwiched between a glass plate and a PET film or the like, as well as laminated glass in which an interlayer film is sandwiched between two glass plates, is included. The laminated glass is a laminate including a glass plate, and it is preferred that at least one glass plate be used. It is preferred that each of the first lamination glass member and the second lamination glass member be a glass plate or a PET film, and the laminated glass include a glass plate as at least one of the first lamination glass member and the second lamination glass member. It is especially preferred that both of the first and second lamination glass members be glass plates.

Examples of the glass plate include a sheet of inorganic glass and a sheet of organic glass. Examples of the inorganic glass include float plate glass, heat ray-absorbing plate glass, heat ray-reflecting plate glass, polished plate glass, figured glass, wired plate glass, green glass, and the like. The organic glass is synthetic resin glass substituted for inorganic glass. Examples of the organic glass include a polycarbonate plate, a poly(meth)acrylic resin plate, and the like. Examples of the poly(meth)acrylic resin plate include a polymethyl (meth)acrylate plate, and the like.

The thickness of each of the first lamination glass member and the second lamination glass member is preferably 1 mm or more, and is preferably 5 mm or less, more preferably 3 mm or less. Moreover, when the lamination glass member is a glass plate, the thickness of the glass plate is preferably 0.5 mm or more, more preferably 0.7 mm or more, and is preferably 5 mm or less, more preferably 3 mm or less. When the lamination glass member is a PET film, the thickness of the PET film is preferably 0.03 mm or more and is preferably 0.5 mm or less.

The method for producing the laminated glass is not particularly limited. First, the interlayer film is sandwiched between the first lamination glass member and the second lamination glass member to obtain a laminate. Then, for example, by passing the obtained laminate through pressure rolls or subjecting the obtained laminate to decompression suction in a rubber bag, the air remaining between the first and the second lamination glass members and the interlayer film is removed. Then, the laminate is preliminarily bonded together at about 70°C to 110°C to obtain a preliminarily press-bonded laminate. Next, by putting the preliminarily press-bonded laminate into an autoclave or by pressing the laminate, the laminate is press-bonded at about 120°C to 150°C and under a pressure of 1 MPa to 1.5 MPa. In this way, laminated glass can be obtained.

Each of the interlayer film and the laminated glass can be used for automobiles, railway vehicles, aircraft, ships, and buildings and the like. Each of the interlayer film and the laminated glass can also be used for applications other than these applications. It is preferred that the interlayer film and the laminated glass be an interlayer film and laminated glass for vehicles or for buildings respectively, and it is more preferred that the interlayer film and the laminated glass be an interlayer film and laminated glass for vehicles respectively. Each of the interlayer film and the laminated glass can be used for a windshield, side glass, rear glass, roof glass or glass for backlight of an automobile, and the like. The interlayer film and the laminated glass are suitably used for automobiles. The interlayer film is suitably used for obtaining laminated glass for an automobile.

Hereinafter, the present invention will be described in more detail with reference to examples and comparative examples. The present invention is not limited only to these examples.

In polyvinyl acetal resins used, n-butyraldehyde which has 4 carbon atoms is used for the acetalization. With regard to the polyvinyl acetal resin, the acetalization degree (the butyralization degree), the acetylation degree and the content of the hydroxyl group were measured by a method conforming to JIS K6728 "Testing methods for polyvinyl butyral". In this connection, even in the cases of being measured according to ASTM D1396-92, numerical values similar to those obtained by a method in accordance with JIS K6728 "Testing methods for polyvinyl butyral" were exhibited.

The following materials were prepared.

### (Thermoplastic resin)

Polyvinyl acetal resin (polyvinyl butyral resin, average polymerization degree of 1700, content of hydroxyl group of 30% by mole, acetylation degree of 1% by mole, acetalization degree (butyralization degree) of 69% by mole))
Polyvinyl acetal resin (polyvinyl butyral resin, average polymerization degree of 3000, content of hydroxyl group of 22% by mole, acetylation degree of 13% by mole, acetalization degree (butyralization degree) of 65% by mole))
Polyvinyl acetal resin (polyvinyl butyral resin, average polymerization degree of 1700, content of hydroxyl group of 30.5% by mole, acetylation degree of 1% by mole, acetalization degree (butyralization degree) of 68.5% by mole))

### (Plasticizer)

Triethylene glycol di-2-ethylhexanoate (3GO)

### (Ultraviolet absorber)

Ultraviolet absorber (X):
Ultraviolet absorber represented by the above formula (X11) ("Tinuvin 234" available from BASF Japan Ltd.)
Ultraviolet absorber represented by the above formula (X12) ("Tinuvin 640" available from BASF Japan Ltd.)
Ultraviolet absorber represented by the above formula (X13) ("Eversorb 88" available from Everlight Chemical)

Ultraviolet absorber not corresponding to ultraviolet absorber (X):
2-(2'-hydroxy-5'-tert-octylphenyl)benzotriazole ("Tinuvin 329" available from BASF Japan Ltd.)
2-(5-tert-butyl-2 hydroxyphenyl)benzotriazole ("Tinuvin PS" available from BASF Japan Ltd.)
2,2',4,4'-tetrahydroxybenzophenone ("SEESORB 106" available from SHIPRO KASEI KAISHA, LTD., an ultraviolet absorber not having a benzotriazole skeleton but having a benzophenone skeleton)

### (Metal salt)

Metal salt 1: Mixture (50 : 50 (weight ratio)) of magnesium 2-ethylbutyrate and magnesium acetate
Metal salt 2: Potassium acetate

### (Oxidation inhibitor)

BHT (2,6-di-t-butyl-p-cresol)

### (Example 1)

Preparation of composition for forming interlayer film:
The following ingredients were mixed, and kneaded sufficiently with a mixing roll to obtain a composition for forming an interlayer film.

Polyvinyl butyral resin (average polymerization degree: 1700, content of hydroxyl group: 30% by mole, acetylation degree: 1% by mole, acetalization degree (butyralization degree): 69% by mole): 100 parts by weight
Triethylene glycol di-2-ethylhexanoate (3GO): 40 parts by weight
Ultraviolet absorber represented by the above formula (X12): 0.4 parts by weight
Metal salt 1 in an amount that gives a magnesium amount of 0.038 parts by weight (metal salt 1 in an amount that gives a magnesium amount in the obtained interlayer film of 60 ppm)
Oxidation inhibitor (BHT) in an amount of 0.2% by weight in the obtained interlayer film

### Preparation of interlayer film:

By extruding a composition for forming an interlayer film with an extruder, a single-layered interlayer film (thickness: 760 um) having only the first layer was prepared.

### Preparation of laminated glass:

The obtained interlayer film was sandwiched between two sheets of 2.5-mm thick clear glass (300 mm long × 300 mm wide) conforming to JIS R3202:1996 to obtain a laminate. The obtained laminate was put into a rubber bag and the inside thereof is degassed for 20 minutes with a degree of vacuum of 2.6 kPa, after which the laminate in the degassed condition was transferred into an oven, and vacuum-pressed by retention at 90°C for 30 minutes, and thus the laminate was preliminarily press-bonded. The preliminarily press-bonded laminate was subjected to press-bonding for 20 minutes under conditions of 135°C and a pressure of 1.2 MPa in an autoclave to obtain a laminated glass. The obtained laminated glass corresponds to the aforementioned laminated glass A.

### (Examples 2 to 6 and Comparative Examples 1 to 9)

A single-layered interlayer film (thickness: 760 um) was prepared in the same manner as that in Example 1 except that the kinds and the contents of the ultraviolet absorbers, and the kinds and the contents of the metal salts were changed to those shown in Tables 2 to 4. Regarding the oxidation inhibitor, the oxidation inhibitor of the same kind and the same blending amount as those in Example 1 was used.

### (Example 7)

Preparation of resin composition for forming first layer:
The following ingredients were mixed, and kneaded sufficiently with a mixing roll to obtain a composition for forming a first layer.

Polyvinyl butyral resin (average polymerization degree: 3000, content of hydroxyl group: 22% by mole, acetylation degree: 13% by mole, acetalization degree: 65% by mole): 100 parts by weight
Triethylene glycol di-2-ethylhexanoate (3GO): 40 parts by weight
Ultraviolet absorber represented by the above formula (X12): 0.4 parts by weight
Metal salt 1 in an amount that gives a magnesium amount of 0.038 parts by weight (metal salt 1 in an amount that gives a magnesium amount in the obtained first layer of 60 ppm)
Oxidation inhibitor (BHT) in an amount of 0.2% by weight in the obtained first layer

Preparation of resin composition for forming second layer and third layer:
The following ingredients were mixed, and kneaded sufficiently with a mixing roll to obtain a resin composition for forming a second layer and a third layer.

Polyvinyl butyral resin (average polymerization degree: 1700, content of hydroxyl group: 30.5% by mole, acetylation degree: 1% by mole, acetalization degree: 68.5% by mole): 100 parts by weight
Triethylene glycol di-2-ethylhexanoate (3GO): 40 parts by weight
Ultraviolet absorber represented by the above formula (X12): 0.4 parts by weight
Metal salt 1 in an amount that gives a magnesium amount of 0.038 parts by weight (metal salt 1 in an amount that gives a magnesium amount in the obtained second and third layers of 60 ppm)
Oxidation inhibitor (BHT) in an amount of 0.2% by weight in the obtained second layer and third layers

### Preparation of interlayer film:

By coextruding the resin composition for forming a first layer and the resin composition for forming a second layer and a third layer using a coextruder, an interlayer film (760 um in thickness) having a three-layer structure (second layer/first layer/third layer) was obtained.

### Preparation of laminated glass:

Laminated glass was obtained in the same manner as that in Example 1 except that the obtained interlayer film was used. The obtained laminated glass corresponds to the aforementioned laminated glass A.

### (Examples 8, 9)

An interlayer film having three-layered structure (second layer/first layer/third layer) (thickness: 760 um) was prepared in the same manner as that in Example 7 except that the kinds and the contents of the ultraviolet absorbers, and the kinds and the contents of the metal salts were changed to those shown in Table 5. Regarding the oxidation inhibitor, the oxidation inhibitor of the same kind and the same blending amount as those in Example 7 was used.

### (Evaluation)

### (1) Maximum value of transmittance at a wavelength of 300 nm or more and 350 nm or less of interlayer film

By measuring transmittance at a wavelength of 300 nm or more and 350 nm or less of the obtained laminated glass (laminated glass A) using a spectrophotometer ("U-4150" available from Hitachi High-Tech Corporation) according to the above-described method, the maximum value of transmittance at a wavelength of 300 nm or more and 350 nm or less of the interlayer film was determined.

### (2) Transmittance at a wavelength of 400 nm of the interlayer film

By measuring transmittance at a wavelength of 400 nm of the obtained laminated glass (laminated glass A) using a spectrophotometer ("U-4150" available from Hitachi High-Tech Corporation) according to the above-described method, transmittance at a wavelength of 400 nm of the interlayer film was determined.

### (3) Ultraviolet transmittance Tuv of interlayer film

By measuring transmittance at a wavelength of 300 nm or more and 400 nm or less of the obtained laminated glass (laminated glass A) using a spectrophotometer ("U-4150" available from Hitachi High-Tech Corporation) according to the above-described method, ultraviolet transmittance Tuv of the interlayer film was determined.

### [Criteriafor judgement in ultraviolet transmittance Tuv of interlayer film]

∘: Ultraviolet transmittance Tuv of interlayer film is 0.5% or less.
×: Ultraviolet transmittance Tuv of interlayer film is more than 0.5%

### (4) Yellow index YI of interlayer film

By measuring total light transmittance of the obtained laminated glass (laminated glass A) using a spectrophotometer ("U-4150" available from Hitachi High-Tech Corporation) according to the above-described method, yellow index YI of the interlayer film was determined.

### (5) Reactivity between ultraviolet absorber and metal salt (absolute value of ΔYI)

A variation (ΔYI) in yellow index YI of interlayer film depending on the presence and absence of a metal salt was determined between interlayer films containing the same kind of ultraviolet absorber. In other words, ΔYI is a value obtained by subtracting yellow index YI of a referential interlayer film (interlayer film not containing a metal salt) from yellow index YI of an interlayer film to be evaluated. More specifically, ΔYI is a value determined by the following formula. Combination of the interlayer film to be evaluated and the referential interlayer film is as follows. The closer to 0 the absolute value of ΔYI, the lower the reactivity between the ultraviolet absorber and the metal salt is suppressed to, meaning that yellowing is less likely to occur in the interlayer film. ΔYI = (YI of interlayer film to be evaluated) - (YI of referential interlayer film)

More specific description is as follows. ΔYI = (YI of interlayer film obtained in Examples 1 to 4) - (YI of interlayer film obtained in Comparative Example 1) ΔYI = (YI of interlayer film obtained in Example 5) - (YI of interlayer film obtained in Comparative Example 2) ΔYI = (YI of interlayer film obtained in Example 6) - (YI of interlayer film obtained in Comparative Example 3) ΔYI = (YI of interlayer film obtained in Example 7) - (YI of interlayer film obtained in Comparative Example 1) ΔYI = (YI of interlayer film obtained in Example 8) - (YI of interlayer film obtained in Comparative Example 2) ΔYI = (YI of interlayer film obtained in Example 9) - (YI of interlayer film obtained in Comparative Example 3) ΔYI = (YI of interlayer film obtained in Example 4) - (YI of interlayer film obtained in Comparative Example 5) ΔYI = (YI of interlayer film obtained in Example 6) - (YI of interlayer film obtained in Comparative Example 7) ΔYI = (YI of interlayer film obtained in Example 8) - (YI of interlayer film obtained in Comparative Example 0)

### [Criteria for judgement in reactivity between ultraviolet absorber and metal salt (absolute value of ΔYI)]

○: Absolute value of ΔYI is 0.1 or less
×: Absolute value of ΔYI is more than 0.1

### (6) Adhesivity between interlayer film and lamination glass member (measurement of pummel value)

The obtained laminated glass was left to stand for 16 hours in an environment at a temperature of -18°C ± 0.6°C, and a center part (part of 150 mm long × 150 mm wide) of the laminated glass after standing was hit with a hammer with a head part of 0.45 kg, and crushed until the grain diameter of the glass was 6 mm or less. The degree of exposure of the film after the glass was partially peeled was measured, and a pummel value was determined according to the following Table 1. A pummel value is a value for investigating the degree of adhesive force between the interlayer film and the glass plate, and is defined by the degree of exposure (area %) of the film after partial peeling of the glass, and is defined in Table 1. The case when the obtained pummel value was 2 to 7 was evaluated as "o", and other cases were evaluated as "×".

**[Table 1]**

| Degree of exposure (area %) of interlayer film | Pummel value |
|---|---|
| 90 < degree of exposure ≤ 100 | 0 |
| 85 < degree of exposure ≤ 90 | 1 |
| 60 < degree of exposure ≤ 85 | 2 |
| 40 < degree of exposure ≤ 60 | 3 |
| 20 < degree of exposure ≤ 40 | 4 |
| 10 < degree of exposure ≤ 20 | 5 |
| 5 < degree of exposure ≤ 10 | 6 |
| 2 < degree of exposure ≤ 5 | 7 |
| Degree of exposure ≤ 2 | 8 |

Configurations of interlayer films and results are shown in the following Tables 2 to 5.

**[Table 2]**

| | | | | Example 1 | Example 2 | Example 3 | Example 4 | Comparative Example 1 |
|---|---|---|---|---|---|---|---|---|
| Interlayer film | Number of layers | | - | 1 | 1 | 1 | 1 | 1 |
| | Thermoplastic resin | Kind | - | PVB | PVB | PVB | PVB | PVB |
| | | Content | parts by weight | 100 | 100 | 100 | 100 | 100 |
| | Plasticizer | Kind | - | 3GO | 3GO | 3GO | 3GO | 3GO |
| | | Content | parts by weight | 40 | 40 | 40 | 40 | 40 |
| | Ultraviolet absorber | Kind | - | Tinuvin640 | Tinuvin640 | Tinuvin640 | Tinuvin640 | Tinuvin640 |
| | | Content | parts by weight | 0.4 | 0.8 | 0.4 | 0.4 | 0.4 |
| | Metal salt 1 (Magnesium salt) | Content of metal | ppm | 60 | 60 | 30 | 0 | 0 |
| | | | parts by weight | 0.038 | 0.038 | 0.019 | 0 | 0 |
| | Metal salt 2 (Potassium salt) | Content of metal | ppm | 0 | 0 | 30 | 60 | 0 |
| | | | parts by weight | 0 | 0 | 0.019 | 0.038 | 0 |
| | Weight ratio (content of metal contained in metal salt/content of ultraviolet absorber) | | - | 9.50 | 4.75 | 9.50 | 9.50 | 0.00 |
| | Thickness of interlayer film | | µm | 760 | 760 | 760 | 760 | 760 |
| Evaluation | Transmittance of interlayer film | Maximum value at a wavelength of 300 nm or more and 350 nm or less | % | 0.017 | 0.017 | 0.05 | 0.02 | 0.08 |
| | | Wavelength 400 nm | % | 67.4 | 11.6 | 66.6 | 67.7 | 64.8 |
| | Ultraviolet transmittance Tuv of interlayer film | | % | 0.0863 | 0.0108 | 0.0550 | 0.0477 | 0.0204 |
| | | | Judgement | ○ | ○ | ○ | ○ | ○ |
| | Yellow index YI of interlayer film | | - | 0.2968 | 0.3511 | 0.3100 | 0.3236 | 0.3425 |
| | Reactivity between ultraviolet absorber and metal salt (\|ΔYI\|) | | - | 0.046 | 0.009 | 0.033 | 0.019 | Criteria |
| | | | Judgement | ○ | ○ | ○ | ○ | |
| | Adhesivity between interlayer film and lamination glass member (measurement of pummel value) | | - | ○ | ○ | ○ | ○ | × |

**[Table 3]**

| | | | | Example 5 | Comparative Example 2 | Example 6 | Comparative Example 3 |
|---|---|---|---|---|---|---|---|
| Interlayer film | Number of layers | | - | 1 | 1 | 1 | 1 |
| | Thermoplastic resin | Kind | - | PVB | PVB | PVB | PVB |
| | | Content | parts by weight | 100 | 100 | 100 | 100 |
| | Plasticizer | Kind | - | 3GO | 3GO | 3GO | 3GO |
| | | Content | parts by weight | 40 | 40 | 40 | 40 |
| | Ultraviolet absorber | Kind | - | Tinuvin234 | Tinuvin234 | Eversorb88 | Eversorb88 |
| | | Content | parts by weight | 0.4 | 0.4 | 0.4 | 0.4 |
| | Metal salt 1 (Magnesium salt) | Content of metal | ppm | 60 | 0 | 60 | 0 |
| | | | parts by weight | 0.038 | 0 | 0.038 | 0 |
| | Metal salt 2 (Potassium salt) | Content of metal | ppm | 0 | 0 | 0 | 0 |
| | | | parts by weight | 0 | 0 | 0 | 0 |
| | Weight ratio (content of metal contained in metal salt/content of ultraviolet absorber) | | - | 9.50 | 0.00 | 9.50 | 0.00 |
| | Thickness of interlayer film | | µm | 760 | 760 | 760 | 760 |
| Evaluation | Transmittance of interlayer film | Maximum value at a wavelength of 300 nm or more and 350 nm or less | % | 0.08 | 0.03 | 0.017 | 0.06 |
| | | Wavelength 400 nm | % | 70.6 | 70.8 | 19.9 | 36.3 |
| | Ultraviolet transmittance Tuv of interlayer film | | % | 0.0839 | 0.0761 | 0.0077 | 0.0037 |
| | | | Judgement | ○ | ○ | ○ | ○ |
| | Yellow index YI of interlayer film | | - | 0.2381 | 0.2007 | 0.6179 | 0.6201 |
| | Reactivity between ultraviolet absorber and metal salt (\|ΔYI\|) | | - | 0.037 | Criteria | 0.002 | Criteria |
| | | | Judgement | ○ | | ○ | |
| | Adhesivity between interlayer film and lamination glass member (measurement of pummel value) | | - | ○ | × | ○ | × |

**[Table 4]**

| | | | | Comparative Example 4 | Comparative Example 5 | Comparative Example 6 | Comparative Example 7 | Comparative Example 8 | Comparative Example 9 |
|---|---|---|---|---|---|---|---|---|---|
| Interlayer film | Number of layers | | - | 1 | 1 | 1 | 1 | 1 | 1 |
| | Thermoplastic resin | Kind | - | PVB | PVB | PVB | PVB | PVB | PVB |
| | | Content | parts by weight | 100 | 100 | 100 | 100 | 100 | 100 |
| | Plasticizer | Kind | - | 3GO | 3GO | 3GO | 3GO | 3GO | 3GO |
| | | Content | parts by weight | 40 | 40 | 40 | 40 | 40 | 40 |
| | Ultraviolet absorber | Kind | - | Tinuvin329 | Tinuvin329 | TinuvinPS | TinuvinPS | SEESORB106 | SEESORB106 |
| | | Content | parts by weight | 0.4 | 0.4 | 0.4 | 0.4 | 0.4 | 0.4 |
| | Metal salt 1 (Magnesium salt) | Content of metal | ppm | 60 | 0 | 60 | 0 | 60 | 0 |
| | | | parts by weight | 0.038 | 0 | 0.038 | 0 | 0.038 | 0 |
| | Metal salt 2 (Potassium salt) | Content of metal | ppm | 0 | 0 | 0 | 0 | 0 | 0 |
| | | | parts by weight | 0 | 0 | 0 | 0 | 0 | 0 |
| | Weight ratio (content of metal contained in metal salt/content of ultraviolet absorber) | | - | 9.50 | 0.00 | 9.50 | 0.00 | 9.50 | 0.00 |
| | Thickness of interlayer film | | µm | 760 | 760 | 760 | 760 | 760 | 760 |
| Evaluation | Transmittance of interlayer film | Maximum value at a wavelength of 300 nm or more and 350 nm or less | % | 0.01 | 0.05 | 0.06 | 0.09 | 0.09 | 0.05 |
| | | Wavelength 400 nm | % | 83.0 | 82.8 | 84.1 | 84.6 | 2.2 | 13.9 |
| | Ultraviolet transmittance Tuv of interlayer film | | % | 0.6802 | 0.6287 | 0.9951 | 0.9647 | 0.0046 | -0.0128 |
| | | | Judgement | × | × | × | × | ○ | ○ |
| | Yellow index YI of interlayer film | | - | 0.2416 | 0.0971 | 0.2279 | 0.0686 | 37.8970 | 2.2949 |
| | Reactivity between ultraviolet absorber and metal salt (\|ΔYI\|) | | - | 0.144 | Criteria | 0.159 | Criteria | 35.602 | Criteria |
| | | | Judgement | × | | × | | × | |
| | Adhesivity between interlayer film and lamination glass member (measurement of pummel value) | | - | ○ | × | ○ | × | ○ | × |

**[Table 5]**

| | | | | Example 7 | Example 8 | Example 9 |
|---|---|---|---|---|---|---|
| Number of layers | | | - | 3 | 3 | 3 |
| First layer | Thermoplastic resin | Kind | - | PVB | PVB | PVB |
| | | Content | parts by weight | 100 | 100 | 100 |
| | Plasticizer | Kind | - | 3GO | 3GO | 3GO |
| | | Content | parts by weight | 40 | 40 | 40 |
| | Ultraviolet absorber | Kind | - | Tinuvin640 | Tinuvin234 | Eversorb88 |
| | | Content | parts by weight | 0.4 | 0.4 | 0.4 |
| | Metal salt 1 (Magnesium salt) | Content of metal | ppm | 60 | 60 | 60 |
| | | | parts by weight | 0.038 | 0.038 | 0.038 |
| | Metal salt 2 (Potassium salt) | Content of metal | ppm | 0 | 0 | 0 |
| | | | parts by weight | 0 | 0 | 0 |
| | Weight ratio (content of metal contained in metal salt/content of ultraviolet absorber) | | - | 9.50 | 9.50 | 9.50 |
| Second and third layers | Thermoplastic resin | Kind | - | PVB | PVB | PVB |
| | | Content | parts by weight | 100 | 100 | 100 |
| | Plasticizer | Kind | - | 3GO | 3GO | 3GO |
| | | Content | parts by weight | 40 | 40 | 40 |
| | Ultraviolet absorber | Kind | - | Tinuvin640 | Tinuvin234 | Eversorb88 |
| | | Content | parts by weight | 0.4 | 0.4 | 0.4 |
| | Metal salt 1 (Magnesium salt) | Content of metal | ppm | 60 | 60 | 60 |
| | | | parts by weight | 0.038 | 0.038 | 0.038 |
| | Metal salt 2 (Potassium salt) | Content of metal | ppm | 0 | 0 | 0 |
| | | | parts by weight | 0 | 0 | 0 |
| | Weight ratio (content of metal contained in metal salt/content of ultraviolet absorber) | | - | 9.50 | 9.50 | 9.50 |
| Thickness of interlayer film | | | µm | 760 | 760 | 760 |
| Evaluation | Transmittance of interlayer film | Maximum value at a wavelength of 300 nm or more and 350 nm or less | % | 0.0168 | 0.076 | 0.0175 |
| | | Wavelength 400 nm | % | 67.3 | 70.7 | 20.0 |
| | Ultraviolet transmittance Tuv of interlayer film | | % | 0.0858 | 0.0827 | 0.0710 |
| | | | Judgement | ○ | ○ | ○ |
| | Yellow index YI of interlayer film | | - | 0.2991 | 0.2015 | 0.6184 |
| | Reactivity between ultraviolet absorber and metal salt (\|ΔYI\|) | | - | 0.043 | 0.001 | 0.002 |
| | | | Judgement | ○ | ○ | ○ |
| | Adhesivity between interlayer film and lamination glass member (measurement of pummel value) | | - | ○ | ○ | ○ |

### EXPLANATION OF SYMBOLS

- 1:: First layer
- 1a:: First surface
- 1b:: Second surface
- 2:: Second layer
- 3:: Third layer
- 11, 11A:: Interlayer film
- 21:: First lamination glass member
- 22:: Second lamination glass member
- 31, 31A:: Laminated glass

## Claims

1. An interlayer film for laminated glass having a one-layer or two or more-layer structure,
the interlayer film for laminated glass containing an ultraviolet absorber represented by formula (X) below, and a metal salt: 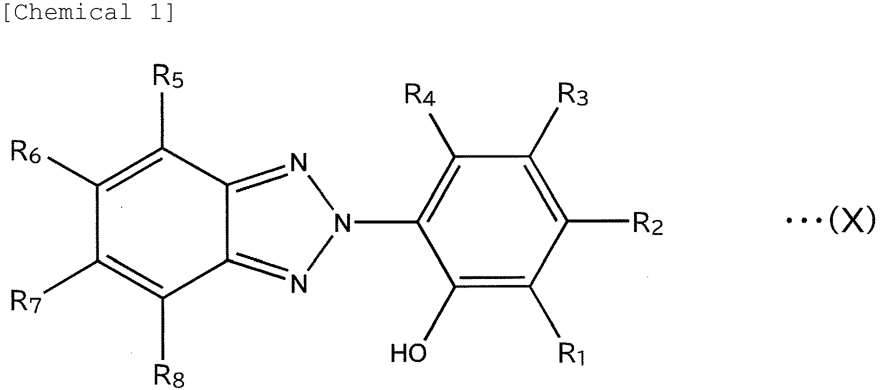 in the formula (X), R₁ representing any group, and R₂ to R₈ each representing a hydrogen atom, an atom other than hydrogen atom, or any group.

2. The interlayer film for laminated glass according to claim 1, comprising a layer containing the ultraviolet absorber and the metal slat.

3. The interlayer film for laminated glass according to claim 1 or 2, wherein in the formula (X), R₁ is an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group.

4. The interlayer film for laminated glass according to any one of claims 1 to 3, wherein the ultraviolet absorber has a molecular weight of 355 or more.

5. The interlayer film for laminated glass according to any one of claims 1 to 4, wherein the ultraviolet absorber includes an ultraviolet absorber represented by formula (X11), formula (X12) or formula (X13) below:

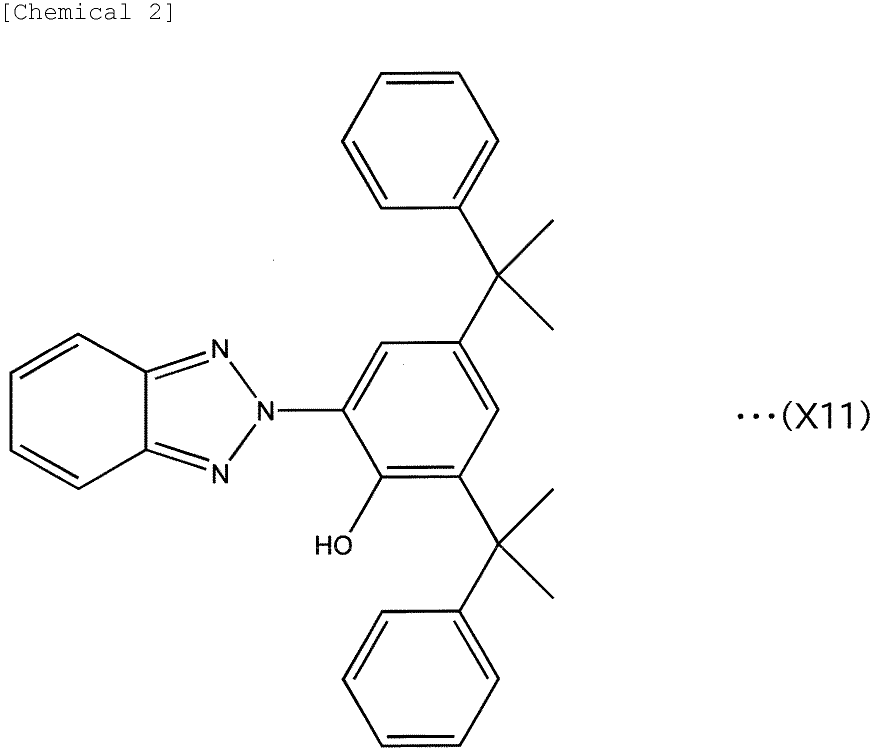

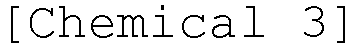

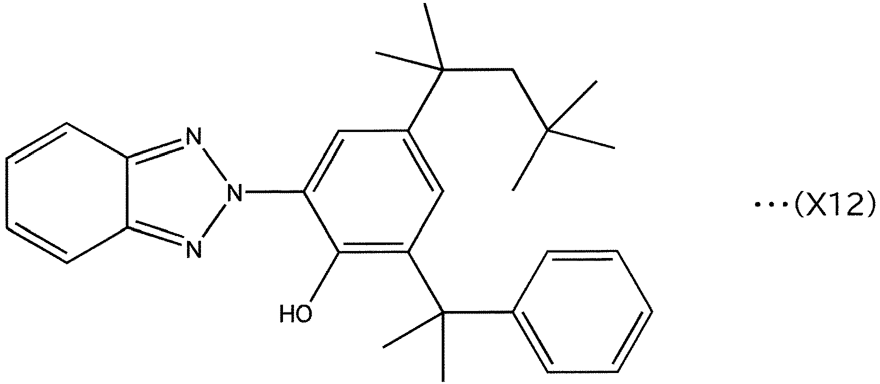

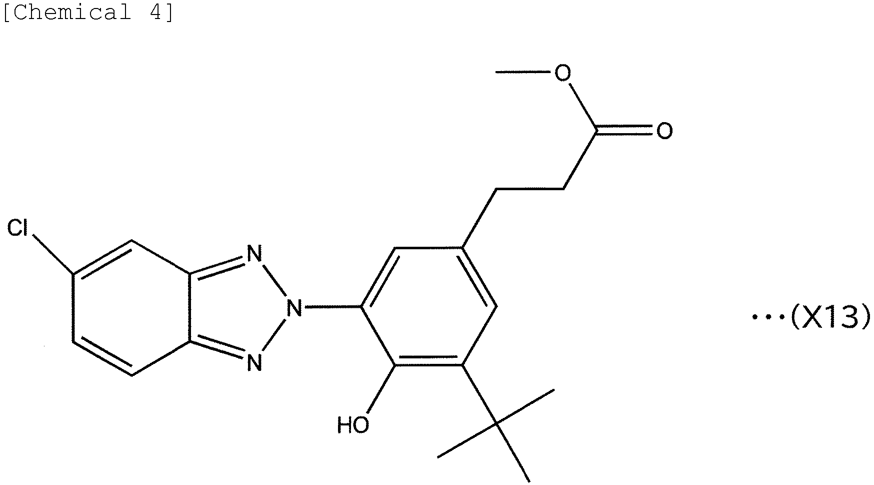

6. The interlayer film for laminated glass according to any one of claims 1 to 5, wherein the metal salt includes an alkali metal salt or an alkali earth metal salt.

7. The interlayer film for laminated glass according to any one of claims 1 to 6, wherein the metal salt includes a magnesium salt of an organic acid having a branched structure.

8. The interlayer film for laminated glass according to any one of claims 1 to 7, wherein the metal salt is a metal salt other than a magnesium salt of an organic acid having a branched structure and includes a metal salt of an organic acid having 2 or more and 8 or less carbon atoms.

9. The interlayer film for laminated glass having a two or more-layer structure, according to any one of claims 1 to 8, comprising a first layer, and a second layer arranged on a first surface side of the first layer.

10. The interlayer film for laminated glass according to claim 9, wherein
the second layer is a surface layer of the interlayer film, and
the second layer contains the ultraviolet absorber and the metal salt.

11. The interlayer film for laminated glass having a three or more-layer structure according to claim 9 or 10, further comprising a third layer arranged on a second surface side opposite to the first surface of the first layer.

12. The interlayer film for laminated glass according to claim 11, wherein
the third layer is a surface layer of the interlayer film, and
the third layer contains the ultraviolet absorber and the metal salt.

13. The interlayer film for laminated glass according to any one of claims 1 to 12, comprising a layer containing the ultraviolet absorber and the metal salt,
wherein in the layer containing the ultraviolet absorber and the metal salt, a weight ratio of a content of metal contained in the metal salt to a content of the ultraviolet absorber is 4 or more and 50 or less.

14. The interlayer film for laminated glass according to any one of claims 1 to 13, wherein the interlayer film has a maximum value of transmittance at a wavelength of 300 nm or more and 350 nm or less of 0.1% or less.

15. The interlayer film for laminated glass according to any one of claims 1 to 14, wherein the interlayer film has ultraviolet transmittance Tuv of 0.5% or less.

16. The interlayer film for laminated glass according to any one of claims 1 to 15, wherein the interlayer film has a transmittance at a wavelength of 400 nm of 1.5% or more.

17. The interlayer film for laminated glass according to any one of claims 1 to 16, wherein an absolute value of difference between yellow index YI of the interlayer film, and yellow index YI of an interlayer film for comparison having a same layer configuration and thickness as the interlayer film except for not containing a metal salt is 0.1 or less.

18. A laminated glass comprising:
a first lamination glass member;
a second lamination glass member; and
the interlayer film for laminated glass according to any one of claims 1 to 17,
the interlayer film for laminated glass being arranged between the first lamination glass member and the second lamination glass member.
